(19) Europäisches Patentamt
European Patent Office
Office européen des brevets

(11) EP 0 670 677 B1

(12) EUROPEAN PATENT SPECIFICATION

(45) Date of publication and mention of the grant of the patent: **26.01.2000 Bulletin 2000/04**

(21) Application number: **94901288.4**

(22) Date of filing: **04.11.1993**

(51) Int. Cl.[7]: **A01N 63/00**, A01N 47/28, C07C 275/16, A01N 63/02 // (A01N63/00, 47:28), (A01N63/02, 63:00)

(86) International application number: **PCT/US93/10671**

(87) International publication number: **WO 94/09630 (11.05.1994 Gazette 1994/11)**

(54) **Potentiator of Bacillus pesticidal activity**

Verstärker für Bazilluspestizide

Potentialisateur de l'activité pesticide de Bacillus

(84) Designated Contracting States: **AT BE CH DE DK ES FR GB GR IE IT LI LU NL PT SE**

(30) Priority: **05.11.1992 US 971786**
**14.12.1992 US 990202**
**20.07.1993 US 95240**

(43) Date of publication of application: **13.09.1995 Bulletin 1995/37**

(83) **Declaration under Rule 28(4) EPC (expert solution)**

(73) Proprietor: **ABBOTT LABORATORIES**
**Abbott Park, Illinois 60064-3500 (US)**

(72) Inventors:
- **MANKER, Denise, Carol**
  **Davis, CA 95616 (US)**
- **LIDSTER, William, David**
  **Sacramento, CA 95825 (US)**
- **STARNES, Robert, Lee**
  **Sacramento, CA 95834 (US)**
- **MACINTOSH, Susan, Caryl**
  **Woodland, CA 95695 (US)**

(74) Representative:
**Modiano, Guido, Dr.-Ing.**
**Modiano, Josif, Pisanty & Staub,**
**Baaderstrasse 3**
**80469 München (DE)**

(56) References cited:

| | |
|---|---|
| **EP-A- 0 339 009** | **EP-A- 0 448 070** |
| **WO-A-92/01383** | **GB-A- 2 000 973** |
| **US-A- 3 911 110** | |

- **JOURNAL OF APPLIED ENTOMOLOGY vol. 101, no. 3 , 1986 pages 304 - 313 H.S.SALAMA ET. AL. 'Possible extension of the activity spectrum of Bacillus thuringiensis strains through chemical additives.'**
- **CHEMICAL ABSTRACTS, vol. 101, no. 15, 8 October 1984, Columbus, Ohio, US; abstract no. 124514u, H.S.SALAMA 'Novel biochemical avenues for enhancing Bacillus thuringiensis endotoxin potency against Spodoptera littoralis (Lep.: Noctuidae)' page 201 ; & ENTOMOPHAGA vol. 29, no. 2 , 1984 pages 171 - 178**
- **FEMS MICROBIOLOGY LETTERS vol. 8, no. 1 , May 1980 pages 1 - 7 P.LÜTHY 'Insecticidal Toxins of Bacillus thuringiensis'**
- **JOURNAL OF ECONOMIC ENTOMOLOGY vol. 81, no. 2 , April 1988 , COLLEGE PARK, MARYLAND US pages 463 - 469 W.A.GARDNER 'Enhanced activity of selected combinations of Bacillus thuringiensis and Beta-exotoxin against Fall Armyworm (Lepidoptera: Noctuidae) larvae'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 670 677 B1

## Description

1. FIELD OF THE INVENTION

The invention is related to a factor which potentiates the pesticidal activity of a *Bacillus* related pesticide, a chemical pesticide and/or a virus with pesticidal properties, as well as methods for obtaining the factor. Furthermore, the invention relates to pesticidal compositions comprising the factor and a pesticidal carrier, or the factor and a *Bacillus* related pesticide, a chemical pesticide and/or a virus with pesticidal properties as well as methods of using such compositions. The invention further relates to a mutant or variant *Bacillus* strain in which such a factor is obtained in larger amounts or has a greater potentiating activity compared to the parental strain and methods for producing such mutant or variant strains.

2. BACKGROUND OF THE INVENTION

Every year, significant portions of the world's commercially important agricultural crops, including foods, textiles, and various domestic plants are lost to pest infestation, resulting in losses in the millions of dollars. Various strategies have been used in attempting to control such pests.

One strategy is the use of broad spectrum pesticides, chemical pesticides with a broad range of activity. However, there are a number of disadvantages to using such chemical pesticides. Because of their broad spectrum of activity, these pesticides may destroy non-target organisms such as beneficial insects and parasites of destructive pests. Additionally, these chemical pesticides are frequently toxic to animals and humans, and targeted pests frequently develop resistance when repeatedly exposed to such substances.

Another strategy has involved the use of biopesticides, which make use of naturally occurring pathogens to control insect, fungal and weed infestations of crops. Biopesticides comprise a bacterium which produces a toxin, a substance toxic to the pest. Biopesticides are generally less harmful to non-target organisms and the environment as a whole than chemical pesticides. The most widely used biopesticide is *Bacillus thuringiensis* (*B.t.*). *B.t.* is a widely distributed, rod shaped, aerobic and spore forming microorganism. During its sporulation cycle, *B.t.* produces a protein(s) in a crystal form known as a crystal delta-endotoxin(s), which kill insect larvae. *B.t.*, therefore, is very useful as an agricultural pesticide.

Some strains, e.g. *Bacillus thuringiensis* subsp. *kurstaki,* and *Bacillus thuringiensis* subsp. *aizawai,* have been found to be specific for Lepidoptera. *Bacillus thuringiensis* subsp. *israelensis* has been found to be specific for Diptera (Goldberg, U.S. Patent No. 4,166,112). Other strains, e.g. *Bacillus thuringiensis* subsp. *tenebrionis* (Krieg et al., 1988, U.S. Patent No. 4,766,203), have been found to be specific for Coleoptera. The isolation of another coleopteran toxic *Bacillus thuringiensis* strain was reported in 1986 (Hernnstadt et al. Bio/Technology vol. 4, 305-308, 1986, US patent 4,764,372, 1988). This strain, designated "*Bacillus thuringiensis* subsp. *san diego*", M-7, has been deposited at the Northern Regional Research Laboratory, USA under accession number NRRL B-15939. However, the assignee of the '372 patent, Mycogen, Corp. has publically acknowledged that *Bacillus thuringiensis* subsp. *san diego* is *Bacillus thuringiensis* subsp. *tenebrionis.* Furthermore, the '372 patent has been assigned to Novo Nordisk A/S. Additionally, there has been disclosed a *B.t.* strain which is toxic against Lepidoptera and Coleoptera (PCT Application No. WO 90/13651). The toxin disclosed in PCT Application No. WO 90/13651 has a molecular weight of 81 kd.

During its sporulation cycle, *B.t.* produces a protein(s) in crystal form known as a crystal delta-endotoxin(s) having a molecular weight ranging from 27-140 kd, which upon ingestion kills insect larvae. Toxic activity may reside in one or more of such crystal proteins in a given *B.t.* strain. Most delta-endotoxins are protoxins that are proteolytically converted into smaller toxic (truncated) polypeptides in the target insect midgut (Höfte and Whiteley, 1989, Microbiol. Rev. 53:242-255). The delta-endotoxins are encoded by *cry* (crystal protein) genes. The *cry* genes have been divided into six classes and several subclasses based on structural similarities and pesticidal specificity. The major classes are Lepidoptera-specific (*cryI*); Lepidoptera-and Diptera-specific (*cryII*); Coleoptera-specific (*cryIII*); Diptera-specific (*cryIV*) (Höfte and Whiteley, 1989, Microbiol. Rev. 53:242-255); Coleoptera- and Lepidoptera-specific (referred to as *cryV* by Tailor et al., 1992, Mol. Microbiol. 6:1211-1217); and Nematode-specific (referred to as *cryV* and *cryVI* by Feitelson et al., 1992, Bio/Technology 10:271-275).

Delta-endotoxins have been produced by recombinant DNA methods. The delta-endotoxins produced by recombinant DNA methods may or may not be in crystal form.

*B.t.* delta-endotoxin is water insoluble except at alkaline pH, and is almost always plasmid encoded. Some strains of *Bacillus thuringiensis* have been shown to produce a heat-stable pesticidal adenine-nucleotide analog, known as β-exotoxin or thuringiensin which is pesticidal alone (Sebesta et al., in H.D. Burges (ed.), Microbial Control of Pests and Plant Diseases, Academic Press, New York p. 249-281, 1981). β-exotoxin has been found in the supernatant of some *Bacillus thuringiensis* cultures. It has a molecular weight of 789 and is comprised of adenosine, glucose, and allaric acid (Lüthy et al., in Kurstak (ed.), Microbial and Viral Pesticides, Marcel Dekker, New York, 1982, pp. 35-72). Its

host range includes, but is not limited to, *Musca domestica*, *Mamestra configurata* Walker, *Tetranychus urticae*, *Drosophila melanogaster*, and *Tetranychus cinnabarinus*. The toxicity of β-exotoxin is thought to be due to inhibition of DNA-directed RNA polymerase by competition with ATP. It has been shown that β-exotoxin is encoded by a Cry plasmid in five *Bacillus thuringiensis* (*B.t.*) strains and that β-exotoxin may be classified as type I or type II β-exotoxin (Levinson et al., 1990, J. Bacteriol. 172:3172-3179). β-exotoxin type I was found to be produced by *B.t.* subsp. *thuringiensis* serotype 1, *B.t.* subsp. *tolworthi* serotype 9, and *B.t.* subsp. *darmstadiensis* serotype 10. β-exotoxin type II was found to be produced by *B.t.* subsp. *morrisoni* serotype 8ab and is active against *Leptinotarsa texana.* Other water soluble factors that have been isolated from *B.t.* include alpha-exotoxin (Luthy, 1980, FEMS Microbiol. Lett. 8:1-7); gamma-exotoxins, which are various proteolytic enzymes including lecithinases, chitinases, and proteases (Forsberg et al., 1976, *Bacillus thuringiensis*: Its Effects on Environmental Quality, National Research Council of Canada, NRC Associate Committee on Scientific Criteria for Environmental Quality, Subcomittees on Pesticides and Related Compounds and Biological Phenomena); sigma exotoxin (Argauer et al., 1991, J. Entomol. Sci. 26:206-213); and anhydrothuringiensin (Coll. Czechoslovak Chem. Comm. 40, 1775, 1975).

The art has strived to achieve increased mortality of *B.t.* formulations. Means have included searching for new strains with increased mortality, attempting to engineer present strains, and attempting to design more effective formulations by combining *B.t.* spores and/or crystals with new pesticidal carriers chemical pesticides, or enhancers (see, for example, U.S. Patent No. 5,250,515, a trypsin inhibitor). It is therefore an object of the present invention to potentiate the pesticidal activity of pesticides.

## 3. <u>SUMMARY OF THE INVENTION</u>

The invention is related to a novel factor which unlike factors known in the art potentiates the pesticidal activity of a *Bacillus* related pesticide. Specifically, the factor of the present invention is a potentiator. As defined herein, a "potentiator" is a substance which has no significant pesticidal activity, e.g. having an $LC_{50}$ ($LC_{50}$, is the concentration of the substance required to kill 50% of the pests) of more than about 3000 μg/g as assayed by bioassay (see Section 6) but acts to increase the pesticidal activity of a *Bacillus* related pesticide at least about 50% and does not cause larval stunting. As noted in Section 2, other substances capable of enhancing pesticidal activity known in the art such as delta-endotoxins, trypsin inhibitors and exotoxins have pesticidal activity.

In a specific embodiment, the factor is water soluble. As defined herein, a substance or compound is "water soluble" if at least about 1 mg of a substance can be dissolved in 1 ml of water. The factor may also potentiate the pesticidal activity of a chemical pesticide and/or a virus with pesticidal properties.

As defined herein, "a *Bacillus* related pesticide" is a *Bacillus* (e.g. *Bacillus thuringiensis* or *Bacillus subtilis*) strain, spore, or substance, e.g. protein or fragment thereof having activity against or which kill pests; a substance that provides plant protection, e.g. antifeeding substance; or a microorganism capable of expressing a *Bacillus* gene encoding a *Bacillus* protein or fragment thereof having activity against or which kill pests (e.g. *Bacillus thuringiensis* delta-endotoxin) and an acceptable carrier (see Section 5.2., *infra*, for examples of such carriers). The pest may be, for example, an insect, a nematode, a mite, or a snail. A microorganism capable of expressing a *Bacillus* gene encoding a *Bacillus* protein or fragment thereof having activity against or which kill pests inhabits the phylloplane (the surface of the plant leaves), and/or the rhizosphere (the soil surrounding plant roots), and/or aquatic environments, and is capable of successfully competing in the particular environment (crop and other insect habitats) with the wild-type microorganisms and provide for the stable maintenance and expression of a *Bacillus* gene encoding a *Bacillus* protein or fragment thereof having activity against or which kill pests. Examples of such microorganisms include but are not limited to bacteria, e.g. genera *Bacillus, Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, Alcaligenes,* and *Clostridium*; algae, e.g. families *Cyanophyceae, Prochlorophyceae, Rhodophyceae, Dinophyceae, Chrysophyceae, Prymnesiophyceae, Xanthophyceae, Raphidophyceae, Bacillariophyceae, Eustigmatophyceae, Cryptophyceae, Euglenophyceae, Prasinophyceae,* and *Chlorophyceae*; and fungi, particularly yeast, e.g. genera *Saccharomyces, Cryptococcus, Kluyveromyces, Sporobolomyces, Rhodotorula,* and *Aureobasidium.*

As defined herein, "pesticidal activity" measures the amount of activity against a pest through killing or stunting of the growth of the pest or protecting the plant from pest infestation.

The invention further relates to a mutant or variant *Bacillus* strain in which such a factor is obtained in large amounts compared to the parental strain as well as methods for obtaining such a mutant or variant.

The invention further relates to pesticidal compositions comprising the factor and a pesticidal carrier as well as the factor and a *Bacillus* related pesticide, chemical pesticide and/or a virus with pesticidal properties.

The invention further relates to methods of using the pesticidal compositions of the present invention. In one embodiment, the invention relates to a method for controlling a pest comprising exposing the pest to a pest-controlling amount of the composition. In another embodiment, the invention relates to a method for decreasing the resistance of a pest to a *Bacillus* related pesticide comprising exposing the pest to a composition comprising the factor and a pesticidal carrier. The invention further relates to a method for potentiating the pesticidal activity of a *Bacillus* related pesticide comprising administering to a pest exposed to a *Bacillus* related pesticide a composition comprising the factor and carrier in amount effective to potentiate the pesticidal activity of a *Bacillus* related pesticide.

The invention is further directed to a method for obtaining "substantially pure" factor of the present invention comprising the steps of

(a) fermentation of a *Bacillus* strain;
(b) recovering the supernatant of (a); and
(c) isolating the factor from the supernatant.

As defined herein a "substantially pure" factor means a factor which contains less than 10% of contaminants, for example, delta-endotoxin protein.

The invention is further directed to a method for identifying the factor of the present invention comprising

(a) fermentation of a strain of *Bacillus*;
(b) recovering the supernatant of the fermentation of (a); and
(c) assaying the supernatant of (b) for potentiation of a *Bacillus* related pesticide.

4. BRIEF DESCRIPTION OF THE FIGURES

Figure 1 schematically shows the general procedure used for purifying Ia.
Figure 2 shows the $^{13}C$ NMR spectrum of Ia.
Figure 3 shows the proton NMR spectrum of Ia.
Figure 4 shows the deduced structure of Ia. Proton ($^{1}H$) and $^{13}C$ shifts are shown in ppm recorded in $D_2O$.
Figure 5 shows a synthetic scheme for obtaining structure Ia.

5. DETAILED DESCRIPTION OF THE INVENTION

The present invention relates to a factor that potentiates the pesticidal activity of a *Bacillus* related pesticide. The factor may have a molecular weight of from about 350 to about 1200 or in a specific embodiment from about 350 to about 700.

The factor of the present invention potentiates the pesticidal activity of a *Bacillus* related pesticide at least about 1.5 fold to optionally about 1000 fold, preferably from about 100 fold to about 400 fold. In a specific embodiment, the factor of the present invention potentiates the pesticidal activity of a *Bacillus thuringiensis* delta-endotoxin including but not limited to a CryI (including CryIA, CryIB, and CryIC), CryII, CryIII, CryIV, CryV, or CryVI protein in full-length form or a proteolytically processed, truncated form, from about 1.5 fold to about 1000 fold. In a most specific embodiment, the factor of the present invention potentiates a *B.t.* delta-endotoxin from about 100 fold to about 400 fold. The factor may also potentiate the pesticidal activity of a chemical pesticide and/or a virus with pesticidal properties.

The factor may also be water soluble, stable in water up to about 100°C for at least 5 minutes, stable when subjected to direct sunlight for at least about 10 hours, and/or stable at a pH of about 2 for about 10 days.

In a specific embodiment, the factor has from 13 to 24 carbons.

In a more specific embodiment, the factor has from 13 to 19 carbons. In a most specific embodiment, the factor has 13 carbons.

In a specific embodiment the factor has the structure (I)

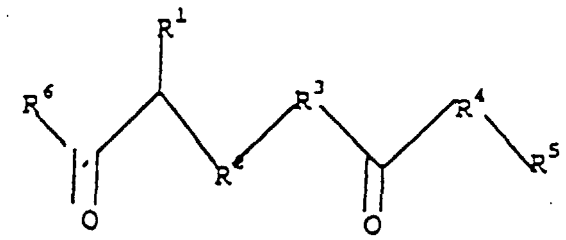

in which

$R^1$ is a amino, hydroxy, $C_{1-10}$-alkyl, benzamide, 3,5-dinitrobenzamide, p-nitrobenzamide, halobenzamide, alkylbenzamide, thiophene amide, furan amide, pyrrole amide, imidazole amide, pyrazole amide, $C_{1-10}$-alkylamino, $C_{1-10}$-dialkylamino, $C_{1-10}$-alkyl or benzyl ester, halogen, or $C_{1-10}$-alkoxy;

$R^2$ is $(CH_2)_n$ wherein n is an integer from 0-10;

$R^3$ and $R^4$ are independently $(NH)_p$ wherein p is 0 or 1;

$R^5$ is

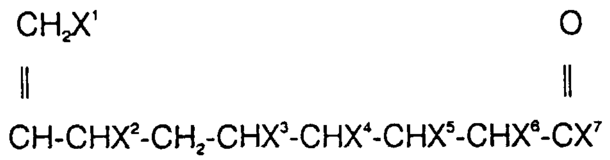

or $(CHOH)_qCH_2OH$ wherein $X^1$, $X^2$, $X^3$, $X^4$, $X^5$, $X^6$ and $X^7$ are independently $NH_2$, OH, acetyl, halogen or $C_{1-10}$-alkoxy and q is an integer from 1-10;

$R^6$ is a hydroxyl, amino, an $C_{1-10}$ alkoxy or benzoxy, benzyl ester, 3,5-dinitrobenzyl ester, p-nitrobenzyl ester, halobenzyl ester, alkylbenzyl ester, thiophene ester, furan ester, pyrrole ester, imidazole ester; or pesticidally acceptable salt thereof including but not limited to phosphate, sulfate, acetate, carbonate, and nitrate.

In a most specific embodiment said factor has the structure

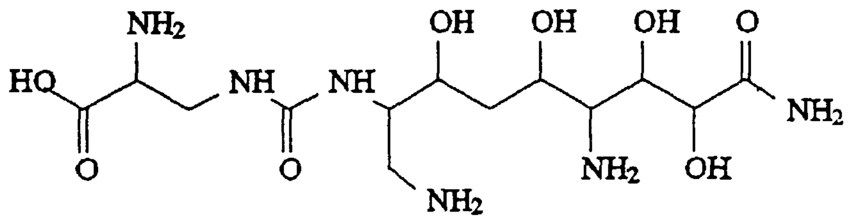

(Ia)

5.1. <u>OBTAINING THE FACTOR</u>

The factor of the present invention may be obtainable from a *Bacillus* fermentation (e.g. *Bacillus subtilis, Bacillus cereus,* and *Bacillus thuringiensis*). In a specific embodiment, the factor of the present invention is obtainable from the supernatant of a *Bacillus thuringiensis* fermentation including *Bacillus thuringiensis* subsp. *kurstaki*, *Bacillus thuringiensis* subsp. *aizawai*, *Bacillus thuringiensis* subsp. *galleriae*, *Bacillus thuringiensis* subsp. *entomocidus*, *Bacillus thuringiensis* subsp. *tenebrionis, Bacillus thuringiensis* subsp. *thuringiensis, Bacillus thuringiensis* subsp. *alesti, Bacillus thuringiensis* subsp. *canadiensis, Bacillus thuringiensis* subsp. *darmstadiensis, Bacillus thuringiensis* subsp. *dendrolimus, Bacillus thuringiensis* subsp. *finitimus, Bacillus thuringiensis* subsp. *kenyae, Bacillus thuringiensis* subsp. *morrisoni, Bacillus thuringiensis* subsp. *subtoxicus, Bacillus thuringiensis* subsp. *toumanoffi* and *Bacillus thuringiensis* subsp. *israelerisis.* In a preferred embodiment, the factor is obtainable from the supernatant of *Bacillus thuringiensis* subsp. *kurstaki*, *Bacillus thuringiensis* subsp. *aizawai,* or *Bacillus thuringiensis* subsp. *galleriae* or mutants thereof having substantially the same potentiating activity. In a specific embodiment, the factor is recovered from a $cry^-$ $spo^-$ mutant of *Bacillus thuringiensis* subsp. *kurstaki.*

In one embodiment, the factor is obtainable from a mutant *Bacillus,* particularly a *Bacillus thuringiensis* in which the factor is produced in larger amounts or a mutant *Bacillus thuringiensis* in which the potentiating activity of the factor obtainable from the mutant is greater, as compared to the parental strain. A "parental strain" as defined herein is the original *Bacillus* strain before mutagenesis. To obtain such mutants, the parental strain may, for example, be treated with a mutagen by chemical means such as N-methyl-N'-nitro-N-nitrosoguanidine or ethyl methanesulfonate, gamma-irradiation, X-ray or UV-irradiation. Specifically, in one method of mutating *Bacillus* strains and selecting such mutants the parental strain is:

i) treated with a mutagen;

ii) the thus treated mutants are grown in a medium suitable for the selection of a mutant strain;

iii) selection of a mutant strain.

According to a preferred embodiment of this method, the selected colonies are grown in a normal production medium, and a final selection for strains capable of increased factor production is performed.

*Bacillus* may be cultured using media and fermentation techniques known in the art (see, for example, Rogoff et al., 1969, J. Invertebrate Path. 14:122-129; Dulmage et al., 1971, J. Invertebrate Path. 18:353-358; Dulmage et al., in Microbial Control of Pests and Plant Diseases, H.D. Burges, ed., Academic Press, N.Y., 1980). Upon completion of the fermentation cycle, the supernatant can be recovered by separating *B.t.* spores and crystals from the fermentation broth by means well known in the art, e.g. centrifugation and/or ultrafiltration. The factor of the present invention is contained in the supernatant which may be recovered by means well known in the art, e.g. ultrafiltration, evaporation, and spray-drying. This procedure is more specifically described in the sections which follow.

Alternatively, the factor of the present invention may be obtained by chemical synthesis using procedures known in the art. As noted above in a specific embodiment, the factor has the structure (Ia)

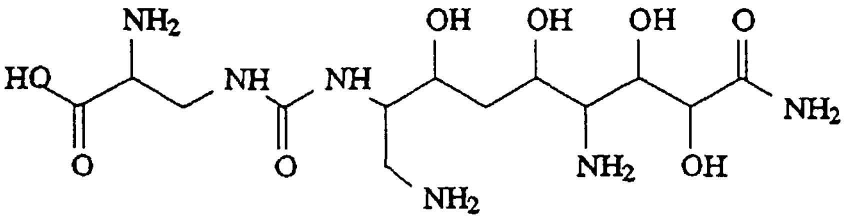

A synthetic scheme is shown in Figure 5. 2,3-Diaminopropionic acid (DAP) is protected using Boc (t-butylcarbamate). The fully N-protected DAP may be converted to the imidizoyl by treatment with diimidazoic carbonate to give compound A (see Figure 5). Compound B (see Figure 5) may be prepared from 3-amino-3-deoxy-aldonic acid by selective substitution of the terminal primary alcohol by using a benzene sulfonyl leaving group. Condensation of A and B will be achieved under Julia conditions (NaH, THF) followed by desulfurization with Raney nickel. The keto group in compound C (see Figure 5) may be stereoselectively reduced by chelation control using sodium tris acetoxyborohydride. Deprotected C may be treated with isocyanate D derived from DAP to give the corresponding ureido derivative which will be deprotected by hydrogenolysis to give compound (Ia).

In one embodiment, the amino groups of (Ia) obtained synthetically or from a *Bacillus* supernatant may be acylated with either acid chloride or acid anhydride in the presence of aqueous sodium hydroxide to obtain triacetamides, tribenzamides and trioctamides of (Ia). Alternatively, the amino and hydroxy groups may be per acylated by first forming methyl esters using excess diazomethane, followed by per acylation on the amino and hydroxyl groups by treatment with an acyl anhydride (e.g. acetic anhydride, benzoic acid anhydride, n-octanoic acid anhydride) in pyridine. In another embodiment, starting with an acylated derivative, a mixed amide ester may be prepared by selective methylation of the carboxyl followed by treatment with an acyl anhydride in pyridine. In another embodiment, the carboxylic acid may be derivatized to form alkyl or aryl esters or amide. In yet another embodiment there may be selective oxidations of the hydroxy substituents to ketone and chain shortened carboxylic acid analogs.

In another embodiment, the amino groups of (Ia) may be protected as t-butyl carbamates by treatment with Boc anhydride in the presence of aqueous sodium carbonate, followed by selective protection of the hydroxyl groups using benzyl bromide in sodium hydride to give (X) shown below

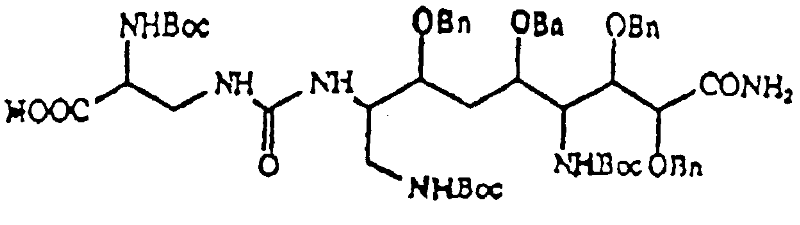

This fully protected derivative may be hydrolyzed at the ureido linkage using aqueous sodium hydroxide. This may be followed by selective acylation with for example amino acids, hydroxy carboxylic acids or dicarboxylic acid including but not limited to aspartic acid, glutamic acid, 2-aminoadipic acid, malic acid, adipic acid in the presence of N,N-dicyclohexylcarbodiimide (DCC). N-Boc and O-benzyl groups may be removed with mild acid cleavage and hydrogenolysis over palladium on charcoal to obtain structure (II) below

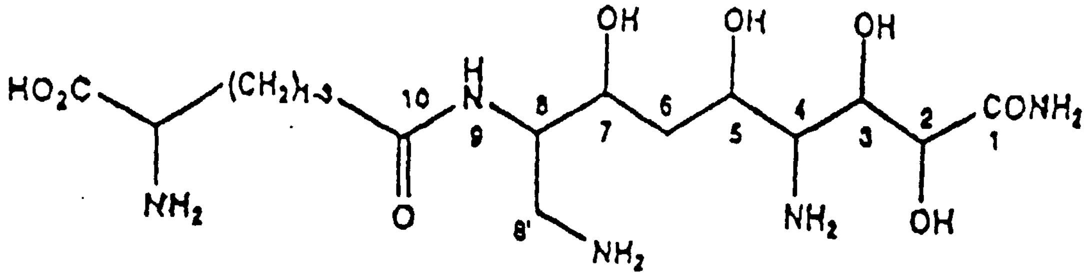

In yet another embodiment, the fully protected compound (X) may be treated with various isocyanates derived from amino acids including but not limited to glycine, alanine, valine, phenylalanine, phenylglycine, methionine, normethionine to obtain 9-ureido derivatives. Protecting groups may be removed as described above to obtain structure (III)

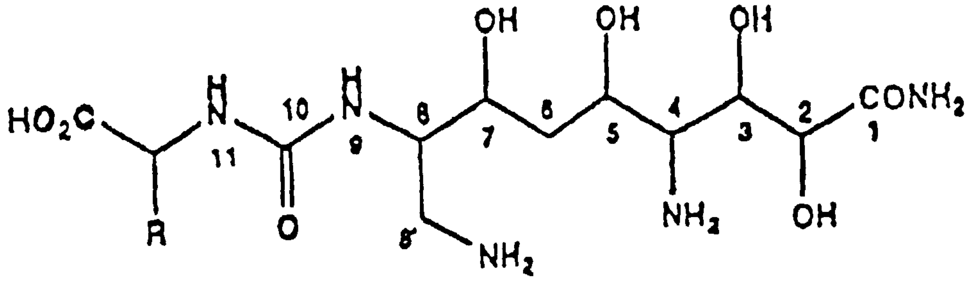

in which R is a naturally occurring amino acid.

In yet another embodiment, 2,3-diaminopropionic acid is protected at the 2-position using Boc in the presence of aqueous sodium carbonate to give N-2-Boc-2,3-diaminopropionic acid. This derivative may be acylated with per acetyl aldonic acids including but not limited to penta-O-acetyl-D-gluconic acid, penta-O-acetyl-D-mannoic acid, penta-O-acetyl-D-galactonic acid, hepta-O-acetyl-D-erythro-L-manno actoanoic acid in the presence of DCC, followed by solvolysis of the acetyl groups and Boc groups in the presence of methanolic ammonia (Chem. Pharm. Bull. 1985, 33:509-514). Polyhydroxy amide analogs having the structure (IV) may be obtained with mild acid treatment

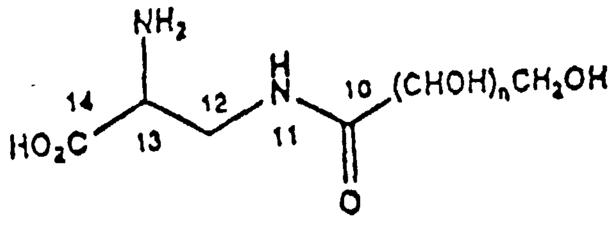

in which n is 1-10. The same sequence may be applied using peracetylated aldaric acids, for example, D-glucaro-6,3-lactone, to yield polyhydroxy with primary terminal carboxamide groups.

In yet another embodiment, a protected derivative of 2,3-diaminopropionic acid is coupled with an isocyanate derived from an amino sugar derivative with a phosgene equivalent. The amino sugar in one embodiment may be a 1- or 2-aminohexose (e.g. 1-amino-1-deoxysorbitol, glucosamine, mannosamine) and has the structure (V)

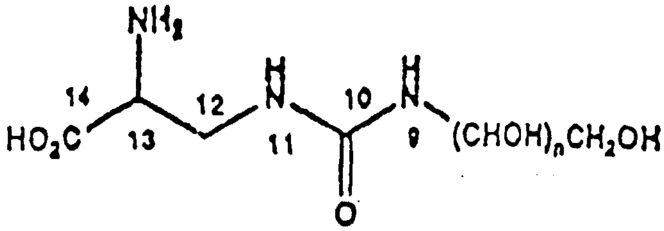

in which n is 1-10.

Purification of the factor of the present invention can be carried out by various procedures known in the art, including but not limited to chromatography (e.g. ion exchange, affinity, and size exclusion column chromatography), electrophoretic procedures, differential solubility, extraction, or any other standard technique known in the art.

The potentiating activity of the factor of the present invention of the pesticidal activity of *Bacillus* related pesticide, virus having pesticidal activity, or chemical pesticide against various pests may be assayed using procedures known in the art, such as an artificial insect diet incorporated, artificial diet overlay, leaf painting, leaf dip, and foliar spray. Specific examples of such assays are given in Section 6, *infra.*

5.2. COMPOSITIONS COMPRISING THE FACTOR

The factor of the present invention described *supra* can be formulated along with a *Bacillus* related pesticide and an acceptable carrier into a pesticidal composition(s) that is for example, a suspension, a solution, an emulsion, a dusting powder, a dispersible granule, a wettable powder, an emulsifiable concentrate, an aerosol or impregnated granule. Examples of such *Bacillus* related pesticides preferably include but are not limited to pesticides produced by *Bacillus thuringiensis* subsp. *kurstaki* (*B.t.k.*) (marketed as DIPEL™ from Abbott Laboratories, Inc., JAVELIN™ from Sandoz, BIOBIT™ from Novo Nordisk A/S, FORAY™ from Novo Nordisk A/S, MVP™ from Mycogen, BACTOSPEINE™ from Novo Nordisk A/S, and THURICIDE™ from Sandoz) and *Bacillus thuringiensis* subsp. *aizawai* (*B.t.a.*) (marketed as FLORBAC™ from Novo Nordisk A/S, and XENTARI™ from Abbott Laboratories, Inc.). Further examples include but are not limited to *Bacillus thuringiensis* subsp. *israelensis* (*B.t.i.*) (marketed as BACTIMOS™ from Nova Nordisk A/S and VECTOBAC™ from Abbott Laboratories, Inc.); *Bacillus sphaericus* (*B. sphr.*) (marketed as SPHERIMOS™ from Nova Nordisk A/S); *Bacillus thuringiensis kurstaki/tenebrionis* (marketed as FOIL™ from Ecogen); *Bacillus thuringiensis aizawai/kurstaki* (*B.t.k./B.t.k.*) (marketed as CONDOR™ from Ecogen and AGREE™ from Ciba-Geigy); and *Bacillus thuringiensis kurstaki/kurstaki* (*B.t.k./B.t.k.*)(marketed as CUTLASS™ from Ecogen). The virus having activity against pests may be a baculovirus, e.g. *Autographa californica* nuclear polyhedrosis virus (NPV), *Syngrapha falcifera* NPV, *Cydia pomonella* GV (granulosis virus), *Heliothis zea* NPV, *Lymantria dispar* NPV, *Orgyia pseudotsugata* NPV, *Spodoptera exigua* NPV, *Neodiprion lecontei* NPV, *Neodiprion sertifer* NPV, *Harrisina brillians* NPV, and *Endopiza viteana* Clemens NPV.

In compositions comprising the factor and a *Bacillus* related pesticide, the factor may be present in the amount of at least about 0.002 g/BIU. In one embodiment, the factor may have structure Ia and/or may be present in an amount of at least about 0.1 g/BIU or 0.05 g factor per g *Bacillus* delta-endotoxin and spore, optionally to about 300 g/BIU or 150 g factor per g *Bacillus* delta-endotoxin and spore, preferably 2 g/BIU or 1 g factor per g Bacillus delta-endotoxin and spore. As defined herein "BIU" is billion international units as determined by bioassay. The bioassay compares the sample to a standard *Bacillus* reference material using *Trichoplusia ni* or other pest as the standard test insect. The potency is determined by dividing the reference standard $LC_{50}$ then multiplying by the reference standard potency.

In another embodiment, the composition may comprise the factor of the present invention in substantially pure form or a supernatant from *Bacillus* in dry, concentrated, or liquid form and a suitable pesticidal carrier, examples of which are disclosed *infra.* This composition may be applied separately to a plant, e.g. transgenic plants. Specifically, the composition may be applied to a plant previously containing a *Bacillus*, e.g. *B.t.* gene. In another embodiment, the composition may be applied to a plant previously exposed to a *Bacillus*, e.g. *B.t.* composition present in the amount of at least about 0.01% up to about 60%.

The composition comprising the factor and a pesticidally acceptable carrier in addition to controlling a pest may also be used to decrease the resistance of a pest to a pesticide. Alternatively, the composition may be used to potentiate a *Bacillus* related pesticide. The composition in one embodiment may be applied at the same time as the pesticide in an amount of at least about 2 g factor/BIU up to optionally about 300 g factor/BIU. In another embodiment, the composition may be applied up to about 24 hours after the pesticide as an adjuvant to extend the efficacy of residual pesticide.

Such compositions disclosed above may be obtained by the addition of a surface active agent, an inert carrier, a preservative, a humectant, a feeding stimulant, an attractant, an encapsulating agent, a binder, an emulsifier, a dye, a U.V. protectant, a buffer, a flow agent, or other component to facilitate product handling and application for particular target pests.

Suitable surface-active agents include but are not limited to anionic compounds such as a carboxylate, for example, a metal carboxylate of a long chain fatty acid; an N-acylsarcosinate; mono or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate or dioctyl succinate. Non-ionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitar fatty acid esters, block copolymers of ethylene oxide and propylene oxide, acetylenic glycols such as 2,4,7,9-tetraethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as an amine oxide of polyoxyethylene alkylamine; an amide-linked amine prepared by the condensation of a carboxylic acid with a di- or polyamine; or a quaternary ammonium salt.

Examples of inert materials include but are not limited to inorganic minerals such as kaolin, phyllosilicates, carbonates, sulfates, phosphates or botanical materials such as cork, powdered corncobs, peanut hulls, rice hulls, and walnut shells.

The compositions of the present invention can be in a suitable form for direct application or as a concentrate or primary composition which requires dilution with a suitable quantity of water or other diluent before application. The pesticidal concentration will vary depending upon the nature of the particular formulation, specifically, whether it is a concentrate or to be used directly. The composition contains 1 to 98% of a solid or liquid inert carrier, and 0 to 50%, preferably 0.1 to 50% of a surfactant. These compositions will be administered at the labeled rate for the commercial product, preferably about .009-4.6 kg/hectare when in dry form and at about 0.012-11.68 liters/hectare when in liquid form.

In a further embodiment, the *Bacillus*, e.g. *B.t.* crystal delta-endotoxin and potentiating factor can be treated prior to formulation to prolong the pesticidal activity when applied to the environment of a target pest as long as the pretreatment is not deleterious to the crystal delta-endotoxin or factor. Such treatment can be by chemical and/or physical means as long as the treatment does not deleteriously affect the properties of the composition(s). Examples of chemical reagents include but are not limited to halogenating agents; aldehydes such as formaldehyde and glutaraldehyde; anti-infectives, such as zephiran chloride; alcohols, such as isopropranol and ethanol; and histological fixatives, such as Bouin's fixative and Helly's fixative (see, for example, Humason, Animal Tissue Techniques, W.H. Freeman and Co., 1967).

The compositions of the invention can be applied directly to the plant by, for example, spraying or dusting at the time when the pest has begun to appear on the plant or before the appearance of pests as a protective measure. Plants to be protected within the scope of the present invention include but are not limited to cereals (wheat, barley, rye, oats, rice, sorghum and related crops), beets (sugar beet and fodder beet), drupes, pomes and soft fruit (apples, pears, plums, peaches, almonds, cherries, strawberries, raspberries, and blackberries), leguminous plants (alfalfa, beans, lentils, peas, soybeans), oil plants (rape, mustard, poppy, olives, sunflowers, coconuts, castor oil plants, cocoa beans, groundnuts), cucumber plants (cucumber, marrows, melons), fibre plants (cotton, flax, hemp, jute), citrus fruit (oranges, lemons, grapefruit, mandarins), vegetables (spinach, lettuce, asparagus, cabbages and other brassicae, carrots, onions, tomatoes, potatoes, paprika), lauraceae (avocados, cinnamon, camphor), deciduous trees and conifers (e.g. linden-trees, yew-trees, oak-trees, alders, poplars, birch-trees, firs, larches, pines), or plants such as maize, turf plants, tobacco, nuts, coffee, sugar cane, tea, vines hops, bananas and natural rubber plants, as well as ornamentals. In both cases, the preferred mode of application is by foliar spraying. It is generally important to obtain good control of pests in the early stages of plant growth as this is the time when the plant can be most severely damaged. The spray or dust can conveniently contain another pesticide if this is thought necessary. In a preferred embodiment, the composition of the invention is applied directly to the plant.

The compositions of the present invention may be effective against pests including but not limited to pests of the order Lepidoptera, e.g. *Achroia grisella, Acleris gloverana, Acleris vaniana, Adoxophyes orana, Agrotis ipsilon, Alabama argillacea, Alsophila pometaria, Amyelois transitella, Anagasta kuehniella, Anarsia lineatella, Anisota senatoria, Antheraea pernyi, Anticarsia gemmatalis, Archips sp., Argyrotaenia sp., Athetis mindara, Bombyx mori, Bucculatrix thurberiella, Cadra cautella, Choristoneura sp., Cochylls hospes, Colias eurytheme, Corcyra cephalonica, Cydia latifer-*

*reanus, Cydia pomonella, Datana integerrima, Dendrolimus sibericus, Desmia funeralis, Diaphania hyalinata, Diaphania nitidalis, Diatraea grandiosella, Diatraea saccharalis, Ennomos subsignaria, Eoreuma loftini, Ephestia elutella, Erannis tilaria, Estigmene acrea, Eulia salubricola, Eupocoellia ambiguella, Eupoecilia ambiguella, Euproctis chrysorrhoea, Euxoa messaria, Galleria mellonella, Grapholita molesta, Harrisina americana, Helicoverpa subflexa, Helicoverpa zea, Heliothis virescens, Hemileuca oliviae, Homoeosoma electellum, Hyphantria cunea, Keiferia lycopersicella, Lambdina fiscellaria fiscellaria, Lambdina fiscellaria lugubrosa, Leucoma salicis, Lobesia botrana, Loxostege sticticalis, Lymantria dispar, Macalla thyrsisalis, Malacosoma sp., Mamestra brassicae, Mamestra configurata, Manduca quinquemaculata, Manduca sexta, Maruca testulalis, Melanchra picta, Operophtera brumata, Orgyia sp., Ostrinia nubilalis, Paleacrita vernata, Papilio cresphontes, Pectinophora gossypiella, Phryganidia californica, Phyllonorycter blancardella, Pieris napi, Pieris rapae, Plathypena scabra, Platynota flouendana, Platynota stultana, Platyptilia carduidactyla, Plodia interpunctella, Plutella xylostella, Pontia protodice, Pseudaletia unipuncta, Pseudoplasia includens, Sabulodes aegrotata, Schizura concinna, Sitotroga cerealella, Spilonota ocellana, Spodoptera sp., Thaurnstopoea pityocampa, Tineola bisselliella, Trichoplusia ni, Udea rubigalis, Xylomyges curialis, Yponomeuta padella; Diptera, e.g. Aedes sp., Andes vittatus, Anastrepha ludens, Anastrepha suspensa, Anopheles barberi, Anopheles quadrimaculatus, Armigeres subalbatus, Calliphora stygia, Calliphora vicina, Ceratitis capitata, Chironomus tentans, Chrysomya rufifacies, Cochliomyia macellaria, Culex sp., Culiseta inornata, Dacus oleae, Delia antigua, Delia platura, Delia radicum, Drosophila melanogaster, Eupeodes corollae, Glossina austeni, Glossina brevipalpis, Glossina fuscipes, Glossina morsitans centralis, Glossina moristans morsitans, Glossina moristans submorsitans, Glossina pallidipes, Glossina palpalis gambiensis, Glossina palpalis palpalis, Glossina tachinoides, Haemagogus equinus, Haematobius irritans, Hypoderma bovis, Hydoderma lineatum, Leucopis ninae, Lucilia cuprina, Lucilia sericata, Lutzomyia longlpaipis, Lutzomyia shannoni, Lycoriella mali, Mayetiola destructor, Musca autumnalis, Musca domestica, Neobellieria sp., Nephrotoma suturalis, Ophyra aenescens, Phaenicia sericata, Phlebotomus sp., Phormia regina, Sabethes cyaneus, Sarcophaga bullata, Scatophaga stercoraria, Stomaxys calcitrans, Toxorhynchites amboinensis, Tripteroides bambusa; Coleoptera, e.g. Leptinotarsa sp., Acanthoscelides obtectus, Callosobruchus chinensis, Epilachna varivestis, Pyrrhalta luteola, Cylas formicarius elegantulus, Listronotus oregonensis, Sitophilus sp., Cyclocephala borealis, Cyclocephala immaculata, Macrodactylus subspinosus, Popillia japonica, Rhizotrogus majalis, Alphitoblus diaperinus, Palorus ratzeburgi, Tenebrio molitor, Tenebrio obscurus, Tribolium castaneum, Tribolium confusum, Tribolius destructor*; *Acari, e.g. Oligonychus pratensis, Panonychus ulmi, Tetranychus urticae*; Hymenoptera, e.g. *Iridomyrmex humilis, Solenopsis invicta*; Isoptera, e.g. *Reticulitermes hesperus, Reticulitermes flavipes, Coptotermes formosanus, Zootermopsis angusticollis, Neotermes connexus, Incisitermes minor, Incisitermes immigrans*; Siphonaptera, e.g. *Ceratophyllus gallinae, Ceratophyllus niger, Nosopsyllus fasciatus, Leptopsylla segnis, Ctenocephalldes canis, Ctenocephalldes felis, Echicnophaga gallinacea, Pulex irritans, Xenopsylla cheopis, Xenopsylla vexabilis, Tunga penetrans*; Tylenchida, e.g. *Melodidogyne incognita, Pratylenchus penetrans.*

The following examples are presented by way of illustration.

## 6. <u>EXAMPLE: CHARACTERIZATION OF Ia</u>

As detailed herein, Ia is recovered and purified. The characterization of Ia is detailed *infra.*

### 6.1. <u>RECOVERY AND PURIFICATION OF Ia</u>

*B. thuringiensis* subsp. *kurstaki* strain EMCC0086 (deposited with the NRRL as B-21147) is fermented for 72 hours at 30°C in a medium comprised of a carbon source such as starch, hydrolyzed starch, or glucose and a nitrogen source such as protein, hydrolyzed protein, or corn steep liquor. The production of Ia is detected at 13 hours into the fermentation. Peak activity is found to be at approximately 30 hours.

Supernatant from a *B. thuringiensis* subsp. *kurstak*i fermentation is recovered by centrifugation and then is clarified by ultrafiltration through a 30 kDa MW-CO membrane using a Rhone Poulenc UF system. The 30 kDa filtration removed any remaining cell debris, crystal delta-endotoxin, spores, and soluble protein greater than 30 kDa molecular mass. The permeate is concentrated 10 fold by evaporation. The permeate is centrifuged and then 0.2µ filtered to further remove insolubles from the broth, leaving a clear broth containing Ia.

The purification of Ia to homogeneity is achieved using a multi-step purification procedure shown schematically in Figure 1. In conjunction with the recovery protocol outlined above, the purification proceeded with a 5 kDa ultrafiltration step. The permeate from the 5 kDa ultrafiltration is adsorbed to a Sulfopropyl (SP) cation exchange resin and eluted with an ammonium acetate solution. The compound is then concentrated approximately 30X by lyophilization, and the salt and other contaminants are removed with a BioRad P2 size exclusion column. The pool from the P2 column is run over a high resolution SP HPLC cation exchange column which yielded a homogeneous compound. The contaminating salt is removed by repeated lyophilization.

Activity is monitored by a *Spodoptera exigua* micro-bioassay, and purity is determined by capillary electrophoresis. Sample consisting of 50 µl of Ia and 50 µl of CryIA(c) protein (15 µg/ml) purified from BIOBIT™ FC (100 µl), is applied to individual wells of a jelly tray containing 500 µl of solidified artificial insect diet. The trays containing the various samples are air dried. Two to four 2nd or early 3rd instar *Spodoptera exigua* are added to the wells containing the dried sample. The wells are sealed with mylar poked with holes and are incubated for 2-3 days at 30°C. Degree of stunting and percent mortality are then recorded. Typically, 5 replicate wells are run for each sample.

6.2. STRUCTURE ELUCIDATION

The active compound is found to be water soluble but is not soluble in organic solvents. It is positively charged and reacted with ninhydrin as evidenced by silica thin layer chromatography. $^{13}C$ and proton NMR of the compound are shown in Figures 2 and 3, respectively. $^{13}C$ NMR experiments revealed the presence of 13 carbons. A DEPT experiment determined that there are three quaternary carbons (C), seven methines (CH), three methylenes ($CH_2$) and no methyl groups ($CH_3$). Using proton coupling experiments such as 1-D decoupling and COSY, one large spin system containing eight carbons is identified. In addition, a smaller spin system consisting of two carbons is present. A two-dimensional carbon proton correlation experiment (HETCOR) enabled assignment of each proton resonance in the molecule to its attached carbon (see Figure 4).

Treatment of the active compound (13 mg) with acetic anhydride in pyridine resulted in the formation of an acetylated derivative which is much less polar. This derivative is purified by HPLC to give 3 mg of pure acetylated derivative. Mass spectroscopy analysis revealed that the derivative has 7 acetates and a molecular weight of 690, which gives a molecular weight of 396 and indicates that an even number of nitrogens are present. Also, fragments of 6 acetates and 5 acetates are detected. High resolution data for 5 and 6 acetate daughter ions are 645.2594 (6 acetates) and 607.2519 (5 acetates) which indicate the following molecular formula for Ia, $C_{13}H_{28}N_6O_8$.

Treatment of the active compound (13 mg) with 6 N HCl gave a derivative which is ninhydrin positive. These results indicate the presence of amide bonds. The derivative had the same $R_f$ value as determined by thin layer chromatography as 2,3-diaminopropionic acid. These results along with NMR data, suggest the presence of 2,3 diaminopropionic acid.

The following structure has been elucidated for Ia:

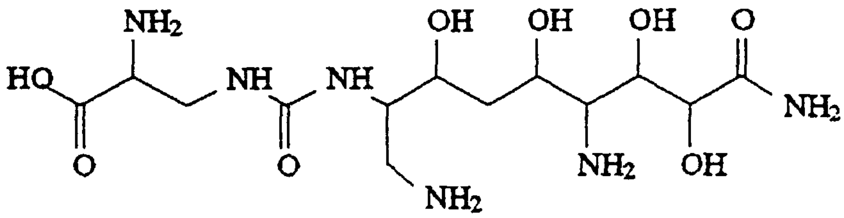

It can be classified as a ureido amide. Constituents include 2 carboxyls (1 amidated), a urea, three aminos, and four hydroxyls. It contains seven chiral centers.

6.3. PROPERTIES OF Ia

The isolated Ia is found to potentiate the activity of *Bacillus thuringiensis* subsp. *kurstaki* and *Bacillus thuringiensis* subsp. *aizawai* crystal delta-endotoxin pesticidal proteins toward *Spodoptera exigua* regardless of the form of the pesticidal proteins. The pesticidal activity of formulated *B.t.k.*, isolated crystals, full-length (130 kDa molecular mass) or truncated CryIA proteins (~65 kDa molecular mass) are all potentiated. The activity of CryII and CryIC inclusions are also potentiated. It is also found to potentiate the activity of the individual truncated CryIA(a), (b), and (c) proteins. Incubation time of Ia with the Cry protein is not found to be critical for bioactivity. However, Ia is inactive alone. The level of potentiation is found to be 100-200 fold for the truncated CryIA proteins, CryII and CryIC inclusions and approximately 320 fold with full-length CryIA(c) (see Tables I and II respectively). Specifically, for full-length protein, 0.75 µg/ml CryIA(c) produced the same insect mortality/stunt score when Ia is included as 240 µg/ml of CryIA(c) alone. In the case of the truncated CryIA(c), an $OD_{280}$ of 0.0006 gave the same stunt score in combination with Ia as the same sample of CryIA(c) tested alone with an $OD_{280}$ of 0.075. CryII inclusions, at a concentration of 0.6 µg/ml gave the same stunt score and similar mortality in combination with Ia as CryII protein alone at 75 µg/ml, a 125 fold potentiation. CryIC inclusions, at 0.3 µg/ml with the addition of Ia gave similar mortality and stunt score as 75 µg/ml of the CryIC protein alone, which reflects a 250 fold level of potentiation. The concentration of CryIA protein that produced stunting yielded mortality on addition of Ia.

Ia is found to be stable upon boiling for 5 minutes, but loses all activity upon autoclaving (>190C). Further, it is stable when subjected to direct sunlight for at least 10 hours. Ia is stable at pH 2 for 3 days, but unstable at pH 12. It is found to lose all activity when exposed to periodic acid or concentrated HCl.

TABLE I

POTENTIATION EFFECTS OF Ia WITH PURIFIED TRUNCATED *Bt* PROTEIN

| *Bt* Protein | | | *Spodoptera Exigua* | |
|---|---|---|---|---|
| Type | OD280 | Ia | Mortality* | Stunt Score† |
| CryIa(a) | 0.055 | - | 0/5 | 2.2 |
| | 0.040 | - | 0/5 | 2.2 |
| | 0.020 | - | 0/5 | 2.0 |
| | 0.020 | + | 2/5 | 0.0 |
| | 0.010 | + | 0/5 | 0.2 |
| | 0.005 | + | 0/5 | 0.0 |
| | 0.0025 | + | 0/5 | 0.4 |
| | 0.0012 | + | 0/5 | 1.8 |
| | 0.0006 | + | 0/5 | 1.6 |
| CryIA(c) | 0.075 | - | 0/5 | 3.4 |
| | 0.040 | - | 0/5 | 2.6 |
| | 0.020 | - | 0/5 | 2.8 |
| | 0.020 | + | 1/5 | 0.0 |
| | 0.010 | + | 0/5 | 0.2 |
| | 0.005 | + | 1/5 | 0.0 |
| | 0.0025 | + | 2/5 | 2.0 |
| | 0.0012 | + | 0/5 | 1.0 |
| | 0.0006 | + | 1/5 | 1.0 |
| None | NA | + | 0/5 | 4.0 |
| None | NA | - | 0/5 | 4.0 |

* Mortality = # insects dead/# total insects after 2 days

† Stunt score is defined by the average size of the live insect larvae at the end of the bioassay: 4.0 = untreated control, 3.0 = 75% size of untreated control, 2.0 = 50% size of untreated control, 1.0 = 25% size of untreated control, 0.0 = no growth or size unchanged from start of experiment.

TABLE II
POTENTIATION EFFECTS OF Ia WITH *Bt* PROTEIN

| *Bt* Protein | | | *Spodoptera Exigua* | |
|---|---|---|---|---|
| Type | μg/ml | Ia | Mortality* | Stunt Score† |
| CryIA(c) | 240 | — | 1/5 | 0.5 |
| | 120 | — | 0/5 | 2.2 |
| | 60 | — | 0/5 | 2.2 |
| | 30 | - | 0/5 | 4.0 |
| | 60 | + | 5/5 | ——— |
| | 30 | + | 5/5 | ——— |
| | 15 | + | 4/5 | 0.0 |
| | 3 | + | 4/5 | 1.0 |
| | 0.8 | + | 2/5 | 1.6 |
| CryII | 300 | — | 1/5 | 0.8 |
| | 150 | — | 2/5 | 0.7 |
| | 75 | — | 1/5 | 0.2 |
| | 38 | - | 0/5 | 0.8 |
| | 19 | - | 0/5 | 1.6 |
| | 9 | - | 0/5 | 1.8 |
| | 5 | - | 1/5 | 4.0 |
| | 38 | + | 3/5 | 1.0 |
| | 19 | + | 2/5 | 0.5 |
| | 9 | + | 3/5 | 0.0 |
| | 5 | + | 1/5 | 0.5 |
| | 2.4 | + | 1/5 | 0.0 |
| | 1.2 | + | 3/5 | 0.5 |
| | 0.6 | + | 2/5 | 0.3 |
| CryII | 300 | — | 2/5 | 0.3 |
| | 150 | — | 2/5 | 0.0 |
| | 75 | — | 1/5 | 0.8 |
| | 38 | - | 0/5 | 3.2 |
| | 38 | + | 5/5 | --- |
| | 19 | + | 5/5 | --- |
| | 9 | + | 5/5 | --- |
| | 5 | + | 4/5 | 0.0 |
| | 2.4 | + | 1/5 | 0.0 |
| | 1.2 | + | 5/5 | --- |
| | 0.6 | + | 3/5 | 1.5 |
| | 0.3 | + | 2/5 | 1.3 |
| None | NA | - | 0/5 | 4.0 |
| None | NA | + | 0/5 | 4.0 |

* Mortality = # insects dead/# total insects after 2 days

† Stunt score is defined by the average size of the live insect larvae at the end of the bioassay: 4.0 = untreated control, 3.0 = 75% size of untreated control, 2.0 = 50% size of untreated control, 1.0 = 25% size of untreated control, 0.0 = no growth or size unchanged from start of experiment.

6.4. EVALUATION OF OTHER SUBSPECIES OF *Bacillus* *thuringiensis* AND OTHER SPECIES OF *Bacilli*

Several *Bacillus* species are evaluated for production of Ia. The strains are fermented for 72 hours at 30°C in a medium comprised of a carbon source such as starch, hydrolyzed starch, or glucose and a nitrogen source such protein, hydrolyzed protein, or corn steep liquor. The supernatants are tested for Ia production using the *Spodoptera exigua* micro-bioassay described *supra. B. thuringiensis* subsp. *aizawai* strain EMCC0087 (deposited with the NRRL as NRRL B-21148) and *B. thuringiensis* subsp. *galleriae* (deposited with the NRRL) are found to produce Ia in about the same concentration as *B. thuringiensis* subsp. *kurstaki.*

Ia is also produced in *B. subtilis, B. cereus, B.t.* subsp. *alesti, B.t.* subsp. *canadiensis, B.t.* subsp. *darmstadiensis, B.t.* subsp. *dendrolimus, B.t.* subsp. *entomocidus, B.t.* subsp. *finitimus, B.t.* subsp. *israelensis, B.t.* subsp. *kenyae, B.t.* subsp. *morrisoni, B.t.* subsp. *subtoxicus, B.t*. subsp. *tenebrionis, B.t.* subsp. *thuringiensis,* and *B.t.* subsp. *toumanoffi, B. cereus, B. subtilis,* and *B. thuringiensis* subsp. *kurstaki* cry- spo- mutant as determined by capillary electrophoresis.

Specifically, a Beckman P/ACE Capillary Electrophoresis System equipped with a 50 µm x 57 cm uncoated capillary, 0.2 M phosphate pH 6.8 buffer, voltage at 15KV, and detection at 200 nm is used for quantifying the level of Ia. Sample volumes are 20 nl with a run time of 25 minutes.

A standard curve is generated using purified Ia as the standard at levels of 1.25 mg/ml, 0.625 mg/ml, 0.3125 mg/ml, 0.156 mg/ml, and 0.078 mg/ml. A linear calibration curve is generated. The resultant $y = mx + b$ equation is used to generate the concentration of Ia in each sample.

Before each run, the capillary is flushed with running buffer (0.2 M phosphate, pH 6.8) for three minutes. After each 25 minute run, the capillary is flushed with 1 N NaOH for 1 minute, filtered HPLC water for 1 minute, 0.5 M phosphoric acid for 3 minutes, and filter HPLC water for 1 minute. The area under each peak is integrated and the peak area is determined and a final concentration is calculated from the standard curve.

6.5. EVALUATION OF *B.t* PRODUCTS

The amount of Ia present in various commercially available *B.t.* products is determined by capillary electrophoresis described in Section 6.4, *supra.* BACTOSPEINE™, JAVELIN™, NOVODOR™, SPHERIMOS™, BACTIMOS™, FORAY™, FLORBAC™ and BIOBIT™ are obtained from Novo Nordisk A/S. XENTARI™ and DIPEL™ are obtained from Abbott Laboratories. AGREE™ is obtained from Ciba-Geigy; MVP™ is obtained from Mycogen and CUTLASS™ is obtained from Ecogen.

The results are shown in Table III, *infra* and indicate that Ia is present in varying quantities ranging from less than 0.001 g Ia/BIU to 0.71 g Ia/BIU.

TABLE III

| Ia IN *Bacillus thuringiensis* PRODUCTS | | | | |
|---|---|---|---|---|
| PRODUCT | type | Lot Number | Potency | Ia g/BIU |
| JAVELIN™ WG | *Btk* | 9942281 | 32000 IU/mg | .071 |
| XENTARI™ | *Bta* | 58715PG | 15000 IU/mg | .06 |
| AGREE™ | *Bta*/*Btk* | RA208004 | 25000 IU/mg | .033 |
| BIOBIT™ HPWP | *Btk* | 5012 | 48950 U/mgPIA | .018 |

TABLE III (continued)

| Ia IN *Bacillus thuringiensis* PRODUCTS | | | | |
|---|---|---|---|---|
| PRODUCT | type | Lot Number | Potency | Ia g/BIU |
| BIOBIT™ FC | *Btk* | AG46669071 | 8 BIU/L | .013 |
| FORAY™ 48B | *Btk* | BBN7018 | 12.6 BIU/L | .012 |
| DIPEL™ | *Btk* | 58739PG | 32,000 IU/mg | .011 |
| FORAY™ 76B | *Btk* | | 20.0 BIU/L | .007 |
| BACTOSPEINE™ | *Btk* | B0B001 | 123653 IU/mg | .003 |
| BACTOSPEINE™ | *Btk* | KX02A | 100,000 IU/mg | .003 |
| BACTOSPEINE™ | *Btk* | WP | 16,000 IU/mg | <.001 |
| NOVODOR™ | *Btt* | 9024 | 16.3 Million LTU/qt | 9.5 x10-9 g/LTU |
| FLORBAC™ | *Bta* | 082-31-1 | 30,000 U/mg E | <.001 |
| SPHERIMOS™ | *B. sphr* | BSN006 | | none |
| MVP™ | *Btk* | 21193542 | | none |
| CUTLASS™ | *Btk*/*Btk* | | | none |
| BACTIMOS™ | *Bti* | BIB0024 | 11,700 IU/mg | none |

6.6. <u>DIET</u> <u>INCORPORATION BIOASSAYS</u>

*B.t.k.* activity is determined by an artificial diet incorporation bioassay using third instar *Spodoptera exigua* larvae, second instar *Helicoverpa zea* larvae, third instar *Spodoptera frugiperda* larvae, second instar *Heliothis virescens* larvae, third instar *Trichoplusia ni* larvae, third instar *Pseudoplusia includens* larvae, third instar *Plutella xylostella* larvae, third instar *Spodoptera littoralis*, and third instar *Mamestra brassicae* larvae.

To determine the level of potentiation by adding Ia to *B.t.* products, and establish the range of insects that are affected, diet incorporation bioassays are performed. In the experiments with high concentrations of Ia against *Spodoptera exigua* (7.4-23.7 g Ia/BIU), purified Ia (70% active ingredient, 30% acetate counter ion) is used to potentiate BIOBIT™ FC (FC represents flowable concentrate). The remaining data presented in Table IV shows the potentiation of BIOBIT™ HPWP (high potency wettable powder) with Ia (0.658% active ingredient). *S. littoralis* and *M. brassicae* are tested using FLORBAC™ HPWP and Ia.

The various *B.t.* products are weighed and Ia is added to give 0.1 to 237 g Ia/BIU. The volume is adjusted with 0.1% Tween™. The samples are sonicated for 1 minute and then diluted to final volume. Neat samples (without Ia) and reference substances are prepared as well. Reference substances include *B.t.k.* HD-1-S-1980 (obtained from the NRRL) which is assigned a potency of 16,000 international units (IU) per milligram and JAVELIN™ WG which has been assigned a potency of 53,000 *Spodoptera* Units/mg (SU).

Standard artificial diet composed of water, agar, sugar, casein, wheat germ, methyl paraben, sorbic acid, linseed oil, cellulose, salts, and vitamins are prepared in a 20 L heated kettle. This provides enough diet to test 10 to 12 samples with seven different concentrations of each test substance. The *B.t.* solutions are serially diluted to give 16 ml aliquots. Each aliquot is added to 184 g of molten diet. The mixture is subsequently homogenized and then poured into a plastic tray bearing 40 individual cells. Three control trays are prepared for each batch of diet. Once the diet has cooled and solidified, one insect of a known age (2-3 instar) is added to each cell, and the trays are covered with a perforated sheet of clear mylar. The trays are placed on racks and incubated for four days at 28°C and 65% relative humidity.

After four days, insect mortality is rated. Each tray is given a sharp blow against a table top, and larvae that did not move are counted as dead. Percent mortality is calculated and the data is analyzed via parallel probit analysis. $LC_{50}$s, $LC_{90}$s, the slope of the regression lines, coefficient of variation, and potencies are estimated. Samples are run a minimum of 3 times or until three potencies are within 20% of a calculated mean for each sample. To calculate the increase in activity associated with each concentration of Ia, the $LC_{50}$ of the *B.t.*/Ia sample is corrected to reflect the amount of *B.t.* in the sample. The $LC_{50}$s of the paired neat samples are divided by the corrected $LC_{50}$ values to give

the fold reduction in $LC_{50}$ associated with Ia.

The following procedure is used to assay for *Lobesia bothrana*. Vine grapes attacked by *Lobesia bothrana* are collected in an unsprayed field and larva is removed. A dilution series of Ia (250 µg/ml, 500 µg/ml, and 1000 µg/ml) is made in water. One larva is put in the middle of the petri dish. If the larva is observed to drink, it is moved into a petri dish with freshly cut grape berries. The larvae are stored at 22°C for 3-4 days.

As shown in Table IV, significant reductions in $LC_{50}$s are observed for all species.

TABLE IV

| Diet Incorporation Bioassays | | |
|---|---|---|
| Insect | g Ia per BIU | Increase in activity Fold reduction in $LC_{50}$ |
| *Spodoptera exigua* (BIOBIT™ HPWP) | 0.1 | 1.5 |
| | 0.2 | 1.7 |
| | 2.0 | 4.3 |
| | 4.0 | 7.5 |
| *Spodoptera exigua* (BIOBIT™ FC) | 7.4 | 13 |
| | 15 | 26 |
| | 30 | 34 |
| | 118 | 59 |
| | 237 | 79 |
| *Spodoptera frugiperda* (BIOBIT™ HPWP) | 0.2 | 2.2 |
| | 0.8 | 3.9 |
| | 2.0 | 7.2 |
| | 4.0 | 11.6 |
| *Trichoplusia ni* (BIOBIT™ HPWP) | 0.1 | 1.1 |
| | 0.2 | 1.2 |
| | 2.0 | 2.0 |
| | 4.0 | 3.1 |
| *Pseudoplusia includens* (BIOBIT™ HPWP) | 0.1 | 0 |
| | 0.2 | 1.2 |
| | 0.8 | 2.1 |
| | 2.0 | 2.4 |
| | 4.0 | 3.4 |
| *Plutella xylostella* (BIOBIT™ HPWP) | 0.2 | 1.6 |
| | 0.8 | 1.3 |
| | 2.0 | 1.4 |
| | 4.0 | 1.9 |
| *Helicoverpa zea* (BIOBIT™ HPWP) | 3.2 | 12.6 |
| *Heliothis virescens* (BIOBIT™ HPWP) | 3.2 | 4.2 |
| *Lobesia bothrana* (BIOBIT™ HPWP) | 2.0 | 3.0 |
| *Spadoptera littoralis* (FLORBAC™ HPWP) | 2.0 | 8.6 |

TABLE IV (continued)

| Diet Incorporation Bioassays | | |
|---|---|---|
| Insect | g Ia per BIU | Increase in activity Fold reduction in $LC_{50}$ |
| *Mamestra brassicae* (FLORBAC™ HPWP) | 2.0 | 4.9 |

The potentiation of various products on *Spodoptera exigua* by Ia is determined using diet incorporation bioassays described *supra.* Amounts of Ia added/BIU product are shown in Table V, *infra.* Ia/B.t. product mixture is incorporated into an agar-based wheat germ casein diet. The insects are placed on the diet for four days and held at 28°C. Mortality is recorded and analyzed using probit analysis. $LC_{50}$, $LC_{90}$ and potency are calculated from matched product lacking Ia. The results shown in Table V indicate that Ia potentiate various *B.t.k.* and *B.t.a.* products obtained from various sources. The *B.t.* strains contained in these products are described in Section 5.2., *supra.*

TABLE V

| Potentiation of *B.t.* Products on *Spodoptera exigua* | | |
|---|---|---|
| Product | g Ia per BIU | Increase in activity Fold reduction in $LC_{50}$ |
| BACTOSPEINE™ WP | 0.4 | 1.04 |
| | 1.7 | 2.3 |
| CONDOR™ | 0.4 | 2.4 |
| | 1.7 | 5.1 |
| AGREE™ | 0.4 | 1.1 |
| | 1.7 | 1.6 |
| CUTLASS™ | 0.4 | 1.1 |
| | 1.7 | 2.5 |
| MVP™ | 0.4 | 6.0 |
| | 1.7 | 7.7 |
| | 2.0 | 12.1 |
| FLORBAC™ HPWP | 0.2 | 1.1 |
| | 0.8 | 2.0 |
| DIPEL™ 2X | 0.2 | 1.2 |
| | 0.8 | 2.3 |
| | 2.0 | 3.9 |
| JAVELIN™ WG | 0.2 | 0 |
| | 0.8 | 1.08 |
| | 2.0 | 2.9 |
| XENTARI™ | 0.2 | 1.2 |
| | 0.8 | 1.6 |
| | 2.0 | 2.4 |

6.7. FOLIAR BIOASSAYS

Foliar bioassays are performed with second instar *Spodoptera exigua* larvae on broccoli plants using BIOBIT™

FC and Ia. The ratio of Ia to BIOBIT™ FC is the same 2 g Ia/BIU BIOBIT™ FC. The treatments are applied to broccoli plants via a track sprayer in a carrier volume of 20 gallons per acre. Leaves are excised from the plants after the spray deposit had dried, and infested with second instar *Spodoptera exigua* larvae. The results are shown in Table VI, *infra.* 100% mortality is observed at a rate of 8.7 BIU/hectare BIOBIT™ FC + Ia, while BIOBIT™ FC alone killed 92% of the larvae at 58.8 BIU/hectare and 8% at 17.6 BIU/hectare. Treated plants are also placed in direct sunlight for eight hours, after which leaves are excised and infested. After eight hours in sunlight, BIOBIT™ FC alone at 58.8 BIU/hectare gave 27% mortality, while BIOBIT™ FC + Ia gave 100% mortality at 8.7 BIU/hectare.

A foliar assay done with early fourth instar larvae had BIOBIT™ FC alone with 75% mortality at 52 BIU/hectare, and BIOBIT™ FC (FC is flowable concentrate) + Ia gave 100% mortality at 13 BIU/hectare.

TABLE VI

| Foliar Bioassays | | | |
|---|---|---|---|
| Treatment | BIU/hectare | % mortality | larval instar |
| BIOBIT™ FC | 58.8 | 92% | 2 |
| BIOBIT™ FC | 17.6 | 8% | 2 |
| BIOBIT™ FC + Ia | 8.7 | 100% | 2 |
| BIOBIT™ FC + 8hr sunlight | 58.8 | 27% | 2 |
| BIOBIT™ FC + Ia + 8hr sunlight | 8.7 | 100% | 2 |
| BIOBIT™ FC | 52 | 75% | 4 |
| BIOBIT™ FC + Ia | 13 | 100% | 4 |

6.8. FIELD TRIALS

Field trials on garbonzo beans (*Spodoptera exigua*) demonstrated that BIOBIT™ FC alone at 70 BIU/hectare gave 51% control while 2 g Ia/BIU BIOBIT™ FC at 40 BIU/hectare provided 89% control (relative to no treatment). JAVELIN™ WG at 45 BIU/hectare gave 51% control.

Field trials on sweet corn (*Spodoptera frugiperda*) demonstrated that at 39.5 BIU/hectare, 2 g Ia/BIU BIOBIT™ FC provided 84% control.

6.9. RESISTANCE RATIOS

Colonies of susceptible and resistant *Plutella xylostella* are bioassayed. Resistant moths are field collected samples from Florida that have developed *B.t.* resistance following intensive exposure to JAVELIN™ WG. BIOBIT™ HPWP with Ia is analyzed using a leaf-dip bioassay. Resistance to JAVELIN™ and XENTARI™ is assayed without Ia. Six cm diameter cabbage leaf disks are dipped for 10 seconds into one of eight different concentrations of *B.t.* products or *B.t.*/Ia formulations. Concentrations range from 1 to 1000 ppm. The leaf disks are allowed to air dry for two hours and placed in plastic petri dishes with second instar (0.2 to 0.4 mg) larvae. Twenty five insects/dose/day are replicated twice to give 50 insects/dose. After 72 hours at 27°C, mortality is recorded. Dose mortality regression is analyzed with probit analysis. Resistance ratios are calculated by dividing the $LC_{50}$ and $LC_{90}$ values of the susceptible moths. The results are shown in Table VII and indicate that the BIOBIT™ HPWP potentiates with 2 g Ia/BIU and 4 g Ia/BIU. Specifically, with 4 g Ia/BIU there is a 2 fold decrease in the $LC_{50}$ resistance ratio and a 10 fold decrease in the $LC_{90}$ resistance ratio

TABLE VII

| *Plutella xylostella* (*B.t.k.* Resistant) Resistance Ratios | | |
|---|---|---|
| PRODUCT TESTED | $LC_{50}$ RR | $LC_{90}$ RR |
| JAVELIN™ WG | 302.6 | 3829.7 |
| BIOBIT™ HPWP | 20.5 | 98.5 |
| 2.0 g Ia/BIU BIOBIT™ HPWP | 23.2 | 88.0 |

TABLE VII (continued)

| *Plutella xylostella* (*B.t.k.* Resistant) Resistance Ratios | | |
|---|---|---|
| PRODUCT TESTED | $LC_{50}$ RR | $LC_{90}$ RR |
| 4.0 g Ia/BIU BIOBIT™ HPWP | 10.4 | 11.5 |
| XENTARI™ | 9.7 | 8.2 |

7. DEPOSIT OF MICROORGANISMS

The following strains of *Bacillus thuringiensis* have been deposited according to the Budapest Treaty in the Agricultural Research Service Patent Culture Collection (NRRL), Northern Regional Research Center, 1815 University Street, Peoria, Illinois, 61604, USA.

| Strain | Accession Number | Deposit Date |
|---|---|---|
| EMCC0086 | NRRL B-21147 | October 6, 1993 |
| EMCC0087 | NRRL B-21148 | October 6, 1993 |

The strains have been deposited under conditions that assure that access to the culture will be available during the pendency of this patent application to one determined by the Commissioner of Patents and Trademarks to be entitled thereto under 37 C.F.R. §1.14 and 35 U.S.C. §122. The deposit represents a substantially pure culture of each deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

## Claims

1. A factor which is obtainable from the supernatant of the fermentation of a *Bacillus* strain and which potentiates the pesticidal activity of a *Bacillus thuringiensis* related pesticide, in which said factor contains 13 carbons, is water soluble, is stable at 100°C for at least 5 minutes, is stable when subjected to ultraviolet light for at least 10 hours, and is stable at a pH of 2 for 3 days.

2. The factor according to claim 1 in which said factor has $^1H$ NMR shifts at 1.5, 3.22, 3.29, 3.35, 3.43, 3.58, 3.73, 3.98, 4.07, 4.15, 4.25, 4.35.

3. The factor according to claim 1 in which the factor has a molecular weight from 350 to 700.

4. The factor according to claim 1 in which the *Bacillus thuringiensis* related pesticide comprises a *Bacillus thuringiensis* strain and an acceptable carrier.

5. The factor according to claim 1 in which the *Bacillus thuringiensis* related pesticide comprises a spore of a *Bacillus thuringiensis* strain and a pesticidally acceptable carrier.

6. The factor according to claim 1 in which the *Bacillus thuringiensis* related pesticide comprises a protein from a *Bacillus thuringiensis* strain or fragment thereof having activity against a pest and a pesticidally acceptable carrier.

7. The factor according to claim 6 in which the protein from a *Bacillus thuringiensis* strain is a *Bacillus thuringiensis* delta-endotoxin.

8. A factor which has the structure (I)

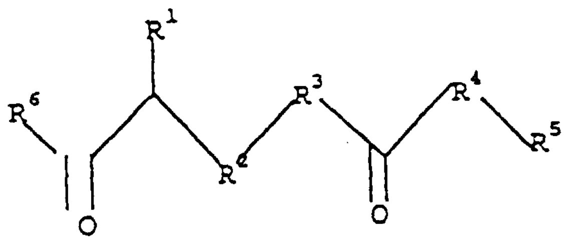

in which $R^1$ is a amino, hydroxy, $C_{1-10}$-alkyl, benzamide, 3,5-dinitrobenzamide, p-nitrobenzamide, halobenzamide, alkylbenzamide, thiophene amide, furan amide, pyrrole amide, imidazole amide, pyrazole amide, $C_{1-10}$-alkylamino, $C_{1-10}$-dialkylamino, $C_{1-10}$-alkyl or benzyl ester, halogen, or $C_{1-10}$-alkoxy;
$R^2$ is $(CH_2)_n$ wherein n is an integer from 0-10;
$R^3$ and $R^4$ are independently $(NH)_p$ wherein p is 0 or 1;
$R^5$ is

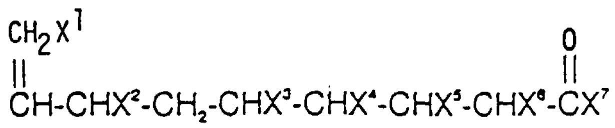

or $(CHOH)_qCH_2OH$ wherein $X^1$, $X^2$, $X^3$, $X^4$, $X^5$, $X^6$ and $X^7$ are independently $NH_2$, OH, acetyl, halogen or $C_{1-10}$-alkoxy and q is an integer from 1-10;
$R^6$ is a hydroxyl, amino, an $C_{1-10}$ alkoxy or benzoxy, benzyl ester, 3,5-dinitrobenzyl ester, p-nitrobenzyl ester, halobenzyl ester, alkylbenzyl ester, thiophene ester, furan ester, pyrrole ester, imidazole ester; or pesticidally acceptable salt thereof.

**9.** The factor according to claim 8 in which the factor has the structure (Ia)

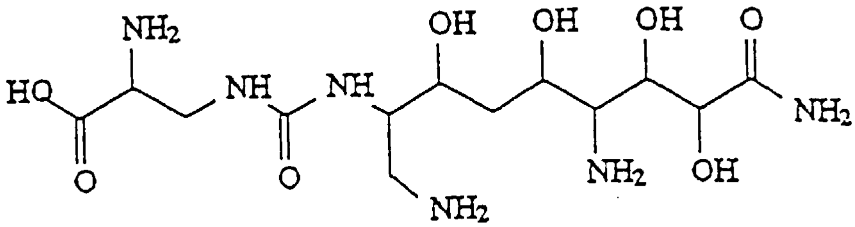

**10.** A factor which potentiates the pesticidal activity of a *Bacillus* related pesticide in which said factor or derivative thereof is obtainable by (a) fermentation of a *Bacillus* strain; (b) recovering the supernatant of the fermentation of (a); and (c) isolating the factor or derivative thereof from the supernatant of (b).

**11.** The factor according to claim 10 in which the factor is obtained from a fermentation supernatant of Bacillus subtilis.

**12.** The factor according to claim 10 in which the factor is obtained from a fermentation supernatant of Bacillus cereus.

**13.** The factor according to claim 10 in which the factor is obtained from a fermentation supernatant of *Bacillus thuringiensis.*

**14.** The factor according to claim 13 in which the factor is obtained from a fermentation supernatant of a subspecies of

a *Bacillus thuringiensis* selected from the group consisting of *Bacillus thuringiensis* subsp. *kurstaki*, *Bacillus thuringiensis* subsp. *aizawai*, *Bacillus thuringiensis* subsp. *galleriae*, or mutants thereof having substantially the same pesticidal activity.

**15.** The factor according to claim 13 in which the factor is obtained from a fermentation supernatant of *Bacillus thuringiensis* subsp. *kurstaki* or mutant thereof having pesticidal activity.

**16.** The factor according to claim 13 in which the factor is obtained from a fermentation supernatant of a cry-spo mutant of *Bacillus thuringiensis* subsp. *kurstaki.*

**17.** A pesticidal composition comprising (a) the factor of claim 1 and (b) a *Bacillus thuringiensis* related pesticide, in which the factor is present in said composition in the amount of at least 0.1 g per BIU.

**18.** A pesticidal composition comprising (a) the factor of claim 1 and (b) a *Bacillus thuringiensis* related pesticide, in which the factor is present in said composition in the amount of at least 0.05 g per g *Bacillus thuringiensis* delta-endotoxin and spore.

**19.** The composition according to claim 17 or 18 in which said composition further comprises a virus having activity against a pest and said factor further potentiates the pesticidal activity of a virus having activity against a pest.

**20.** The composition according to claim 17 or 18 in which the *Bacillus thuringiensis* is selected from the group consisting of *Bacillus thuringiensis* subsp. *kurstaki* and *Bacillus thuringiensis* subsp. *aizawai.*

**21.** A pesticidal composition comprising (a) the factor of claim 1 and (b) a virus having activity against a pest.

**22.** A pesticidal composition comprising (a) the factor of claim 1 and (b) a pesticidal carrier, in which the factor is present in said composition in the amount of at least 0.01 % by weight.

**23.** A method for controlling a pest comprising exposing the pest to a pest-controlling effective amount of the pesticidal composition of claim 17, 18, 21, or 22.

**24.** The method according to claim 23 in which the pest is present on a transgenic plant.

**25.** The method according to claim 24 in which the transgenic plant contains a *Bacillus thuringiensis* gene.

**26.** The method according to claim 24 in which the plant has previously been exposed to a *Bacillus thuringiensis* related pesticide.

**27.** A method for potentiating the pesticidal activity of a *Bacillus thuringiensis* related pesticide comprising administering to a pest exposed to a *Bacillus thuringiensis* related pesticide the composition of claim 22 in an amount effective to potentiate the pesticidal activity of said *Bacillus thuringiensis* related pesticide.

**28.** A method for obtaining the factor of claim 1 comprising

(a) fermentation of a strain of *Bacillus*;
(b) recovering the supernatant of the fermentation of (a); and
(c) isolating the factor from the supernatant of (b) to obtain the substantially pure factor.

**29.** A method for identifying the factor of claim 1 comprising

(a) fermentation of a strain of *Bacillus*;
(b) recovering the supernatant of the fermentation of (a); and
(c) assaying the supernatant of (b) for potentiation of a *Bacillus* related pesticide.

**30.** A mutant or variant of a *Bacillus* strain which produces the factor of claim 1.

**31.** A method for obtaining the mutant or variant of claim 30 comprising

(a) treating the parental strain with a mutagen,

(b) growing the mutated parental strain on a medium suitable for the selection of a mutant strain; and

(c) selection of the mutant strain.

## Patentansprüche

1. Faktor, der aus dem Fermentationsüberstand eines *Bacillus* Stammes erhältich ist und der die pestizide Aktivität eines von *Bacillus Thuringiensis* abgeleiteten Pestizides erhöht, wobei der Faktor 13 Kohlenstoffe enthält, wasserlöslich ist, bei 100°C mindestens 5 Minuten lang stabil ist, gegenüber ultraviolettem Licht mindestens 10 Stunden lang stabil ist, und der bei einem pH-Wert von 2 während 3 Tagen stabil ist.

2. Faktor nach Anspruch 1, wobei der Faktor $^1H$ NMR Verschiebungen bei 1.5, 3.22, 3.29, 3.35, 3.43, 3.58, 3.73, 3.98, 4.07, 4.15, 4.25, 4.35 aufweist.

3. Faktor nach Anspruch 1, wobei der Faktor ein Molekulargewicht von 350 bis 700 besitzt.

4. Faktor nach Anspruch 1, wobei das von *Bacillus Thuringiensis* abgeleitete Pestizid einen *Bacillus Thuringiensis*-Stamm und einen verträglichen Träger umfaßt.

5. Faktor nach Anspruch 1, wobei das von *Bacillus Thuringiensis* abgeleitete Pestizid eine Spore eines *Bacillus Thuringiensis*-Stammes und einen für ein Pestizid verträglichen Träger umfaßt.

6. Faktor nach Anspruch 1, wobei das von *Bacillus Thuringiensis* abgeleitete Pestizid ein Protein eines *Bacillus Thuringiensis*-Stammes oder ein Fragment davon umfaßt, das eine Aktivität gegen einen Schädling besitzt, und einen für ein Pestizid verträglichen Träger umfaßt.

7. Faktor nach Anspruch 6, wobei das Protein des *Bacillus Thuringiensis*-Stammes das *Bacillus Thuringiensis* delta-Endotoxin ist.

8. Ein Faktor, der die Struktur (I):

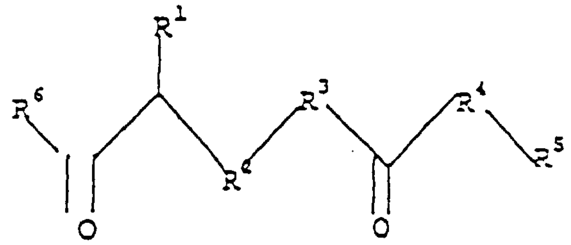

aufweist, worin $R^1$ eine Amino-, Hydroxy-, $C_{1-10}$-Alkyl-, Benzamid-, 3,5-Dinitrobenzamid-, p-Nitrobenzamid-, Halobenzamid-, Alkylbenzamid-, Thiophenamid-, Furanamid-, Pyrrolamid-, Imidazolamid-, Pyrazolamid-, $C_{1-10}$-Alkylamino-, $C_{1-10}$-Dialkylamino-, $C_{1-10}$-Alkyl- oder Benzylester-, Halogen- oder $C_{1-10}$-Alkoxygruppe ist;
$R^2$ $(CH_2)_n$ ist, wobei n eine ganze Zahl von 0-10 ist;
$R^3$ und $R^4$ unabhängig $(NH)_p$ sind, wobei p gleich 0 oder 1 ist;
$R^5$ gleich

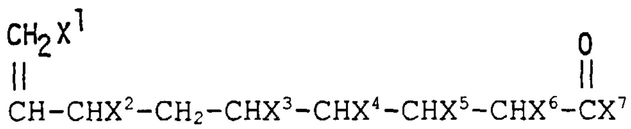

oder $(CHOH)_qCH_2OH$ ist, wobei $X^1$, $X^2$, $X^3$, $X^4$, $X^5$, $X^6$, und $X^7$ unabhängig gleich $NH_2$, OH, Actyl, Halogen

oder $C_{1-10}$-Alkoxy sind, und wobei q eine ganze Zahl von 1-10 ist;

$R^6$ eine Hydroxyl-, Amino-, eine $C_{1-10}$ Alkoxy- oder Benzoxy-, Benzylester-, 3,5-Dinitrobenzylester-, p-Nitrobenzylester-, Halobenzylester-, Alkylbenzylester-, Thiophenester-, Furanester-, Pyrrolester-, Imidazolestergruppe ist; oder ein für ein Pestizid verträgliches Salz davon.

**9.** Faktor nach Anspruch 8, wobei der Faktor die Struktur (Ia) besitzt:

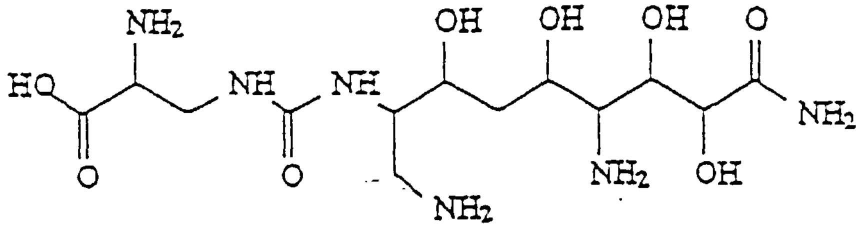

**10.** Faktor, der die pestizide Aktivität eines von *Bacillus* abgeleiteten Pestizids erhöht, wobei der Faktor oder das Derivat davon durch folgende Schritte erhältlich ist: (a) Fermentation eines *Bacillus*-Stammes; (b) Gewinnen des Fermentationsüberstandes von (a); und (c) das Isolieren des Faktors oder der Derivate davon aus dem Überstand von (b).

**11.** Faktor nach Anspruch 10, wobei der Faktor aus einem Fermenationsüberstand von *Bacillus Subtilis* erhalten wird.

**12.** Faktor nach Anspruch 10, wobei der Faktor aus einem Fermentationsüberstand von *Bacillus Cereus* erhalten wird.

**13.** Faktor nach Anspruch 10, wobei der Faktor aus einem Fermentationsüberstand von *Bacillus Thuringiensis* erhalten wird.

**14.** Faktor nach Anspruch 13, wobei der Faktor aus einem Fermentationsüberstand einer Subspezies von einem *Bacillus Thuringiensis* erhalten wird, der aus der Gruppe gewählt ist, die aus *Bacillus Thuringiensis subsp. kurstaki*, *Bacillus Thuringiensis* subsp. *aizawai*, *Bacillus thuringiensis subsp. galleriae* oder Mutanten davon besteht, die im wesentlichen die gleiche pestizide Aktivität besitzen.

**15.** Faktor nach Anspruch 13, wobei der Faktor aus einem Fermentationsüberstand von *Bacillus Thuringiensis subsp. kurstaki* oder Mutante davon, die eine pestizide Aktivität besitzt, erhalten wird.

**16.** Faktor nach Anspruch 13, wobei der Faktor aus einem Fermentationsüberstand einer cry-spo Mutante von *Bacillus Thuringiensis subsp. Kurstaki* erhalten wird.

**17.** Pestizide Zusammensetzung, die (a) den Faktor von Anspruch 1 und (b) ein von *Bacillus Thuringiensis* abgeleitetes Pestizid umfaßt, in dem der Faktor in der Zusammensetzung in der Menge von mindestens 0,1g pro BIU enthalten ist.

**18.** Pestizide Zusammensetzung, die (a) den Faktor von Anspruch 1 und (b) ein von *Bacillus Thuringiensis* abgeleitetes Pestizid umfaßt, wobei der Faktor in der Zusammensetzung in der Menge von mindestens 0,05g pro Gramm *Bacillus Thuringiensis* delta-Endotoxin und Spore vorhanden ist.

**19.** Zusammensetzung nach Anspruch 17 oder 18, wobei die Zusammensetzung weiterhin einen Virus enthält, der eine Aktivität gegen einen Schädling aufweist, und wobei der Faktor weiterhin die pestizide Aktivität eines Virus, der eine Aktivität gegenüber einem Schädling besitzt, verstärkt.

**20.** Zusammensetzung nach Anspruch 17 oder 18 wobei der *Bacillus Thuringiensis* aus der Gruppe gewählt ist, die aus *Bacillus Thuringiensis subsp.* kurstaki und *Bacillus Thuringiensis subsp. Aizawai* besteht.

**21.** Pestizide Zusammensetzung, die (a) den Faktor aus Anspruch 1 und (b) einen Virus, der eine Aktivität gegenüber

einem Schädling besitzt, umfaßt.

**22.** Pestizide Zusammensetzung, die (a) den Faktor aus Anspruch 1 und (b) einen pestiziden Träger umfaßt, wobei der Faktor in der. Zusammensetzung in der Menge von mindestens 0,01 Gewichtsprozent vorhanden ist.

**23.** Verfahren zur Schädlingsbekämpfung, das das Aussetzen des Schädlings mit einer schädlingsbekämpfenden Menge der pestiziden Zusammensetzung nach Anspruch 17, 18, 21 oder 22 umfaßt.

**24.** Verfahren nach Anspruch 23, wobei der Schädling auf einer transgenen Pflanze auftritt.

**25.** Verfahren nach Anspruch 24, wobei die transgene Pflanze ein *Bacillus Thuringiensis* Gen enthält.

**26.** Verfahren nach Anspruch 24, wobei die Pflanze zuvor mit einem von *Bacillus Thuringiensis* abgeleiteten Pestizid ausgesetzt wurde.

**27.** Verfahren zur Potenzierung der pestiziden Aktivität eines von *Bacillus Thuringiensis* abgeleiteten Pestizids, das den Schritt umfaßt, einem Schädling, der einem von *Bacillus Thuringiensis* abgeleiteten Pestizid ausgesetzt war, die Zusammensetzung nach Anspruch 22 in einer Menge zu verabreichen, die wirksam ist, um die pestizide Aktivität des von *Bacillus Thuringiensis* abgeleiteten Pestizids zu verstärken.

**28.** Verfahren zum Erhalt des Faktors nach Anspruch 1, das folgende Schritte umfaßt:

(a) die Fermentation eines *Bacillusstammes*;
(b) das Gewinnen des Fermentationsüberstandes von (a); und
(c) das Isolieren des Faktors aus dem Überstand von (b), um einen im wesentlichen reinen Faktor zu erhalten.

**29.** Verfahren zur Identifizierung des Faktors nach Anspruch 1, das folgende Schritte umfaßt:

(a) die Fermentation eines *Bacillus*-Stammes;
(b) die Gewinnung des Fermentationsüberstandes von (a); und
(c) die Untersuchung des Überstandes von (b) im Hinblick auf die Verstärkung eines von *Bacillus* abgeleiteten Pestizids.

**30.** Eine Mutante oder Variante eines *Bacillus*-Stammes, die den Faktor nach Anspruch 1 produziert.

**31.** Verfahren zur Gewinnung der Mutante oder Variante nach Anspruch 30, das folgende Schritte umfaßt:

(a) das Behandeln des parenteralen Stammes mit einem Mutagen,
(b) das Anzüchten des mutierten parenteralen Stammes auf einem Medium, das für die Auswahl eines Mutantenstammes geeignet ist; und
(c) die Selektion des Mutantenstammes.

**Revendications**

**1.** Facteur qui peut être obtenu à partir du surnageant de la fermentation d'une souche de *Bacillus* et qui potentialise l'activité pesticide d'un pesticide apparenté à *Bacillus thuringiensis*, dans lequel ledit facteur contient 13 carbones, est hydrosoluble, est stable à 100°C pendant au moins 5 min, est stable quand il est soumis à une lumière ultraviolette pendant au moins 10 h et est stable à un pH de 2 pendant 3 jours.

**2.** Facteur selon la revendication 1, dans lequel ledit facteur a des déplacements en RMN $^1H$ à 1,5, 3,22, 3,29, 3,35, 3,43, 3,58, 3,73, 3,98, 4,07, 4,15, 4,25, 4,35.

**3.** Facteur selon la revendication 1, dans lequel le facteur a une masse moléculaire de 350 à 700.

**4.** Facteur selon la revendication 1, dans lequel le pesticide apparenté à *Bacillus thuringiensis* comprend une souche de *Bacillus thuringiensis* et un véhicule acceptable.

**5.** Facteur selon la revendication 1, dans lequel le pesticide apparenté à *Bacillus thuringiensis* comprend une spore

d'une souche de *Bacillus thuringiensis* et un véhicule acceptable du point de vue pesticide.

6. Facteur selon la revendication 1, dans lequel le pesticide apparenté à *Bacillus thuringiensis* comprend une protéine provenant d'une souche de *Bacillus thuringiensis* ou un fragment de celle-ci ayant une activité contre un nuisible et un véhicule acceptable du point de vue pesticide.

7. Facteur selon la revendication 6, dans lequel la protéine provenant d'une souche de *Bacillus thuringiensis* est une delta-endotoxine de *Bacillus thuringiensis.*

8. Facteur qui a la structure (I)

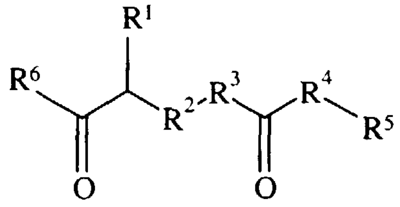

dans laquelle $R^1$ est amino, hydroxyle, alkyle en $C_{1-10}$, benzamide, 3,5-dinitrobenzamide, p-nitrobenzamide, halogénobenzamide, alkylbenzamide, thiophène amide, furane amide, pyrrole amide, imidazole amide, pyrazole amide, alkyle en $C_{1-10}$-amino, dialkyle en $C_{1-10}$-amino, alkyle en $C_{1-10}$ - ou benzylester, halogène ou alcoxy en $C_{1-10}$ ;
$R^2$ est $(CH_2)_n$ où n est un entier de 0 à 10 ;
$R^3$ et $R^4$ sont indépendamment $(NH)_p$ où p est 0 ou 1 ;
$R^5$ est

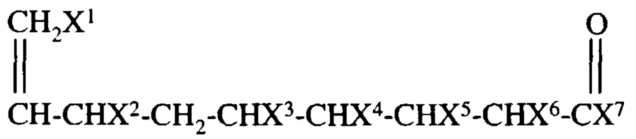

ou $(CHOH)qCH_2OH$ où $X^1$, $X^2$, $X^3$, $X^4$, $X^5$, $X^6$ et $X^7$ sont indépendamment $NH_2$, OH, acétyle, halogène ou alcoxy en $C_{1-10}$ et q est un entier de 1 à 10 ;
$R^6$ est hydroxyle, amino, alcoxy en $C_{1-10}$ ou benzoxy, benzylester, 3,5-dinitrobenzylester, p-nitrobenzylester, halogénobenzylester, alkylbenzylester, thiophène ester, furane ester, pyrrole ester, imidazole ester ; ou sel acceptable du point de vue pesticide de celui-ci.

9. Facteur selon la revendication 8, dans lequel le facteur a la structure (Ia)

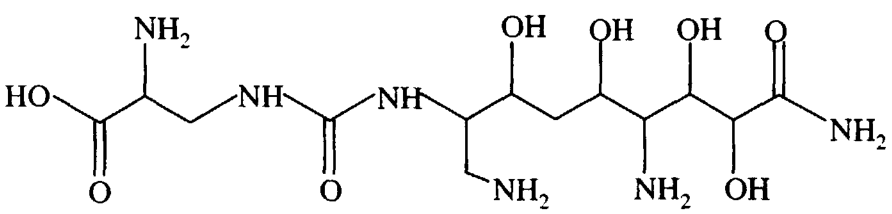

10. Facteur qui potentialise l'activité pesticide d'un pesticide apparenté à *Bacillus* dans lequel ledit facteur ou son dérivé peut être obtenu par (a) fermentation d'une souche de *Bacillus* ; (b) récupération du surnageant de la fermentation de (a) ; et (c) isolement du facteur ou de son dérivé à partir du surnageant de (b).

**11.** Facteur selon la revendication 10, dans lequel le facteur est obtenu à partir d'un surnageant de fermentation de *Bacillus subtilis.*

**12.** Facteur selon la revendication 10, dans lequel le facteur est obtenu à partir d'un surnageant de fermentation de *Bacillus cereus.*

**13.** Facteur selon la revendication 10, dans lequel le facteur est obtenu à partir d'un surnageant de fermentation de *Bacillus thuringiensis.*

**14.** Facteur selon la revendication 13, dans lequel le facteur est obtenu à partir d'un surnageant de fermentation d'une sous-espèce de *Bacillus thuringiensis* choisie dans le groupe consistant en *Bacillus thuringiensis* subsp. *kurstaki*, *Bacillus thuringiensis* subsp. aizawai, *Bacillus thuringiensis* subsp. *galleriae*, ou de leurs mutants ayant sensiblement la même activité pesticide.

**15.** Facteur selon la revendication 13, dans lequel le facteur est obtenu à partir d'un surnageant de fermentation de *Bacillus thuringiensis* subsp. *kurstaki* ou d'un mutant de celle-ci ayant une activité pesticide.

**16.** Facteur selon la revendication 13, dans lequel le facteur est obtenu à partir d'un surnageant de fermentation d'un mutant $cry^{-}spo^{-}$ de *Bacillus thuringiensis* subsp. *kurstaki.*

**17.** Composition pesticide comprenant (a) le facteur selon la revendication 1 et (b) un pesticide apparenté à *Bacillus thuringiensis,* dans laquelle le facteur est présent dans ladite composition en une quantité d'au moins 0,1 g/BIU.

**18.** Composition pesticide comprenant (a) le facteur selon la revendication 1 et (b) un pesticide apparenté à *Bacillus thuringiensis*, dans laquelle le facteur est présent dans ladite composition en une quantité d'au moins 0,05 g/g de delta-endotoxine et de spore de *Bacillus thuringiensis.*

**19.** Composition selon la revendication 17 ou 18, dans laquelle ladite composition comprend en outre un virus ayant une activité contre un nuisible et ledit facteur potentialise en outre l'activité pesticide d'un virus ayant une activité contre un nuisible.

**20.** Composition selon la revendication 17 ou 18, dans laquelle le *Bacillus thuringiensis* est choisi dans le groupe consistant en *Bacillus thuringiensis* subsp. *kurstaki* et *Bacillus thuringiensis* subsp. aizawai.

**21.** Composition pesticide comprenant (a) le facteur selon la revendication 1 et (b) un virus ayant une activité contre un nuisible.

**22.** Composition pesticide comprenant (a) le facteur selon la revendication 1 et (b) un véhicule pesticide, dans laquelle le facteur est présent dans ladite composition en une quantité d'au moins 0,01 % en masse.

**23.** Procédé pour lutter contre un nuisible comprenant l'exposition du nuisible à une quantité efficace pour la lutte contre les nuisibles de la composition pesticide selon la revendication 17, 18, 21 ou 22.

**24.** Procédé selon la revendication 23, dans lequel le nuisible est présent sur une plante transgénique.

**25.** Procédé selon la revendication 24, dans lequel la plante transgénique contient un gène de *Bacillus thuringiensis.*

**26.** Procédé selon la revendication 24, dans lequel la plante a été exposée au préalable à un pesticide apparenté à *Bacillus thuringiensis.*

**27.** Procédé pour potentialiser l'activité pesticide d'un pesticide apparenté à *Bacillus thuringiensis* comprenant l'administration à un nuisible exposé à un pesticide apparenté à *Bacillus thuringiensis* de la composition selon la revendication 22 en une quantité efficace pour potentialiser l'activité pesticide dudit pesticide apparenté à *Bacillus thuringiensis.*

**28.** Procédé pour obtenir le facteur selon la revendication 1 comprenant

(a) la fermentation d'une souche de *Bacillus* ;
(b) la récupération du surnageant de la fermentation de (a) ; et
(c) l'isolement du facteur à partir du surnageant de (b) pour obtenir le facteur sensiblement pur.

**29.** Procédé pour identifier le facteur selon la revendication 1 comprenant

(a) la fermentation d'une souche de *Bacillus* ;
(b) la récupération du surnageant de la fermentation de (a) ; et
(c) la soumission du surnageant de (b) à un essai de potentialisation d'un pesticide apparenté à *Bacillus.*

**30.** Mutant ou variante d'une souche de *Bacillus* qui produit le facteur selon la revendication 1.

**31.** Procédé pour obtenir le mutant ou la variante selon la revendication 30 comprenant

(a) le traitement de la souche-mère avec un mutagène,
(b) la culture de la souche-mère mutée sur un milieu approprié pour la sélection d'une souche mutante, et
(c) la sélection de la souche mutante.

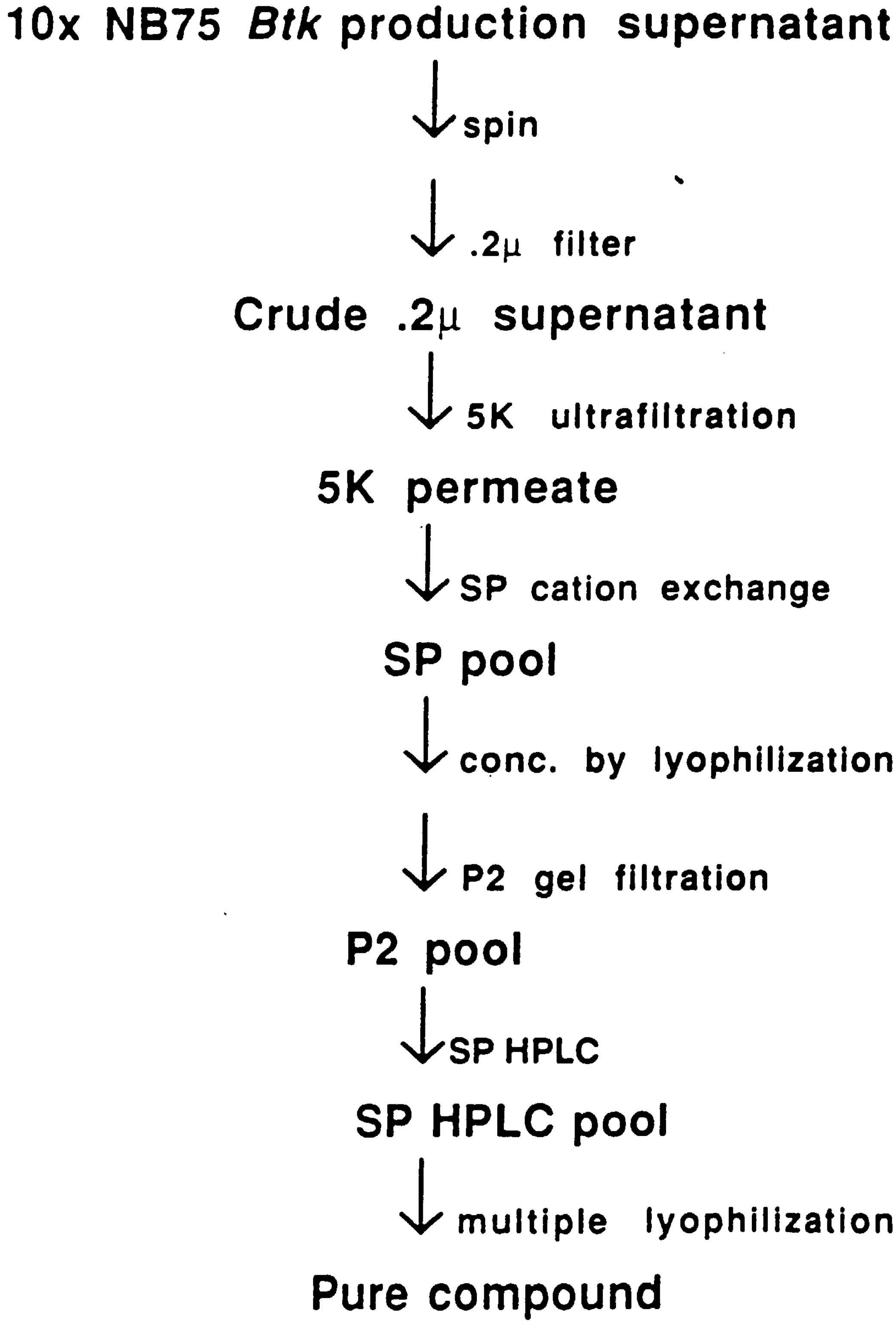
10x NB75 *Btk* production supernatant
spin
.2µ filter
Crude .2µ supernatant
5K ultrafiltration
5K permeate
SP cation exchange
SP pool
conc. by lyophilization
P2 gel filtration
P2 pool
SP HPLC
SP HPLC pool
multiple lyophilization
Pure compound

FIG. 1

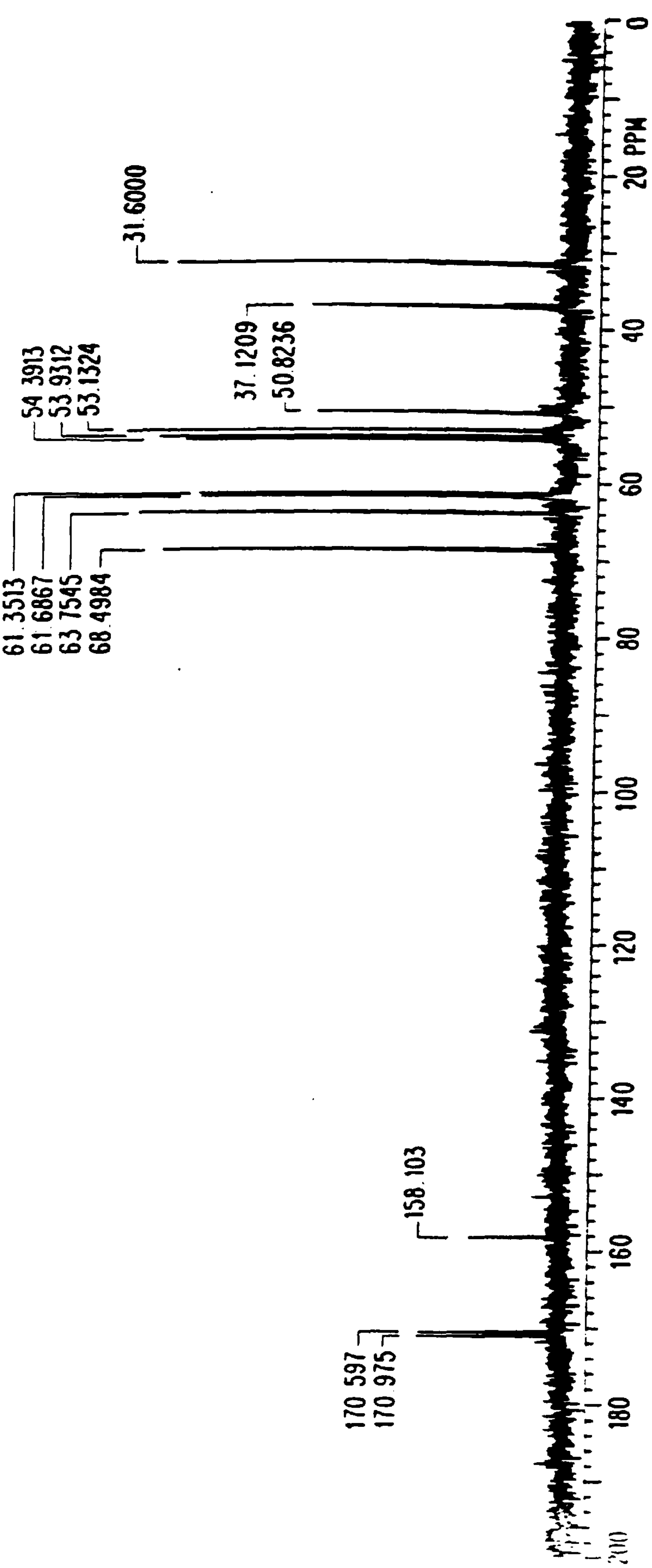
31.6000
37.1209
50.8236
54.3913
53.9312
53.1324
61.3513
61.6867
63.7545
68.4984
158.103
170.597
170.975
0
20 PPM
40
60
80
100
120
140
160
180
200

*FIG.2*

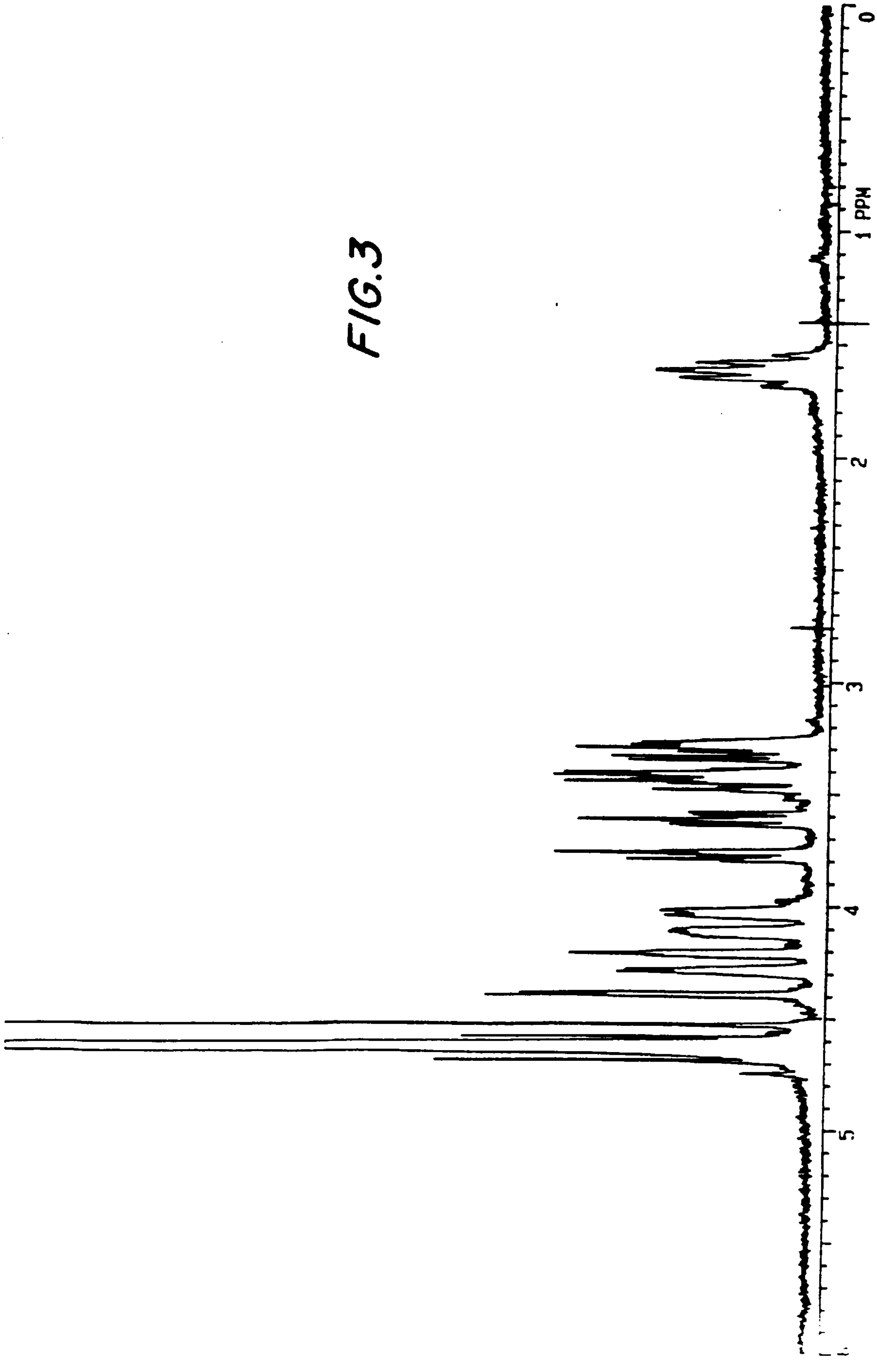


FIG.3

| Position | $^1H$ NMR δ ppm in $D_2O$ | $^{13}C$ NMR δ ppm in $D_2O$ | multiplicity |
|---|---|---|---|
| 1 | | 171.3 | C |
| 2 | 4.35 | 68.6 | CH |
| 3 | 4.01 | 64.1 | CH |
| 4 | 3.35 | 54.4 | CH |
| 5 | 4.15 | 61.8 | CH |
| 6 | 1.5 | 31.6 | $CH_2$ |
| 7 | 3.98 | 61.5 | CH |
| 8 | 3.22 | 53.3 | CH |
| 8' | 3.58, 3.73 | 54.0 | CH2 |
| 10 | | 158.3 | C |
| 12 | 3.29,3.43 | 37.2 | $CH_2$ |
| 13 | 4.25 | 51.1 | CH |
| 14 | | 170.7 | C |

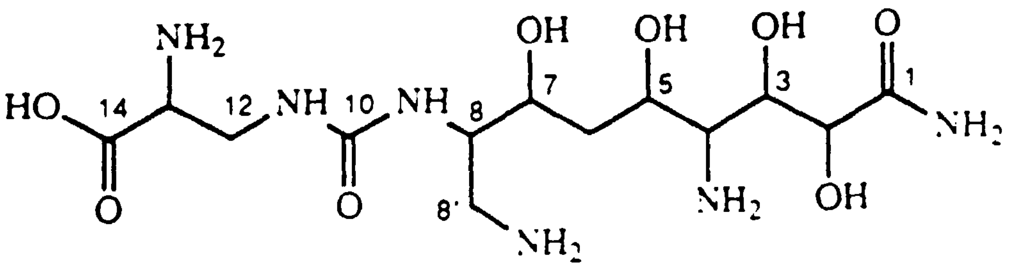

FIG. 4

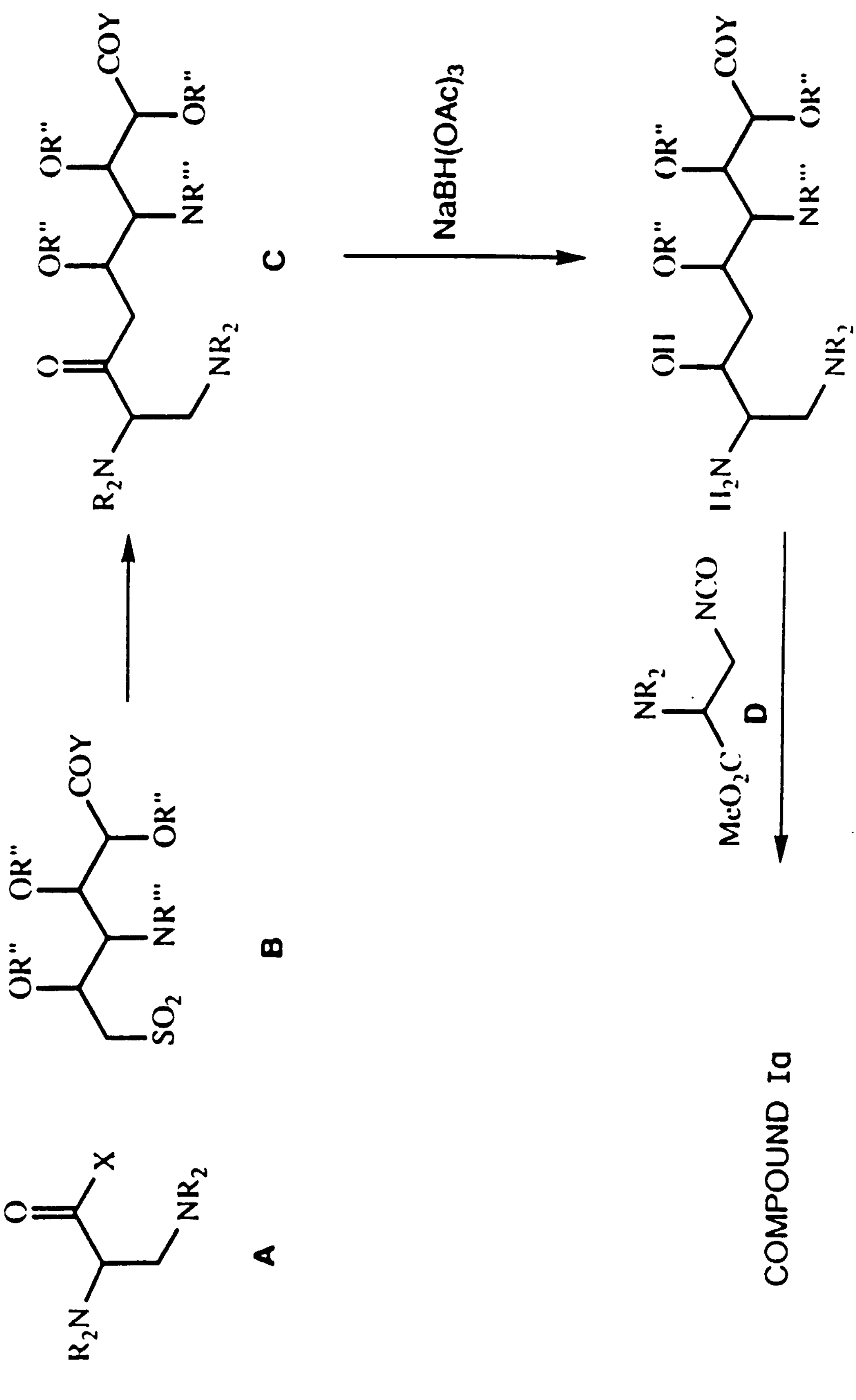


*FIG. 5*